# EUROPÄISCHE PATENTSCHRIFT

(11) **EP 3 067 343 B1**
(45) Veröffentlichungstag und Bekanntmachung des Hinweises auf die Patenterteilung: **25.07.2018**
(21) Anmeldenummer: 15158424.0
(22) Anmeldetag: 10.03.2015
(51) Int. Cl.: C07C 69/732, C08G 65/332, C08L 75/04, C08K 5/134, C08J 9/00, C09K 15/32, C08K 5/1535, C08K 5/526, C08K 5/527, C09K 15/08, C08K 5/00, C08L 71/02

(54) **Antioxidantien zur Herstellung emissionsarmer PUR-Systeme**
Antioxidants for the preparation of low-emission polyurethane systems
Antioxydants destinés à fabriquer des systèmes PUR à faible émission

(43) Veröffentlichungstag der Anmeldung: 14.09.2016
(73) Patentinhaber: Evonik Degussa GmbH, 45128 Essen (DE)
(72) Erfinder: Krebs, Michael, 40219 Düsseldorf (DE); Hubel, Roland, 45136 Essen (DE)

(56) Entgegenhaltungen:
- EP-A1- 0 608 089
- EP-A1- 1 676 887
- EP-A1- 2 500 341
- WO-A1-02/02684
- WO-A1-94/14748
- DE-A1- 2 133 374
- DE-A1- 10 336 883

## Beschreibung

Die vorliegende Erfindung liegt auf dem Gebiet der Antioxidantien sowie auf dem Gebiet der Kunststoffe, insbesondere der Polyurethane. Sie betrifft insbesondere Antioxidationsmittelmischungen sowie Polyurethansysteme, vor allem Polyurethanschäume.

Antioxidantien sind Verbindungen unterschiedlicher chemischer Struktur, die unerwünschte, durch Sauerstoff-Einwirkung und andere oxidative Prozesse bedingte Veränderungen in den zu schützenden Stoffen hemmen oder verhindern sollen.

Sie werden z. B. in Kunststoffen zum Schutz gegen Alterung benötigt, denn synthetische Polymere unterliegen grundsätzlich der Oxidation durch Sauerstoff, wobei oft in geringen Mengen vorliegende Verunreinigungen den Oxidationsprozess unterstützen können. Diese Oxidation kann zu abträglichen Änderungen der mechanischen Eigenschaften des betreffenden Polymers führen und damit des Bauteils, in dem das Polymer seine Verwendung findet. Dies kann letztlich zu einer ungewünschten Funktionsbeeinträchtigung führen. Um solche Oxidationsprozesse zu verhindern oder wenigstens zu inhibieren, werden also Antioxidantien eingesetzt, wie z.B. sterisch gehinderte Phenole.

Dies kann auch bei der Herstellung von Polyurethansystemen, wie z. B. Polyurethanbeschichtungen, Polyurethanadhäsiven, Polyurethandichtmittel, Polyurethanelastomeren oder insbesondere Polyurethanschäumen/-schaumstoffen, erfolgen.

Polyurethansysteme, wie insbesondere -schaumstoffe finden aufgrund ihrer hervorragenden mechanischen und physikalischen Eigenschaften in den verschiedensten Bereichen Verwendung. Einen besonders wichtigen Markt für verschiedenste Typen von PUR-Schäumen (= Polyurethanschäumen), wie konventionelle Weichschäume auf Ether- und Esterpolyolbasis, Kaltschäume (häufig auch als HR-Schäume bezeichnet), Hartschäume, Integralschäume und mikrozellulare Schäume, sowie Schäume deren Eigenschaften zwischen diesen Klassifizierungen liegen, wie z. B. halbharte Systeme, stellt die Automobil- und die Möbelindustrie dar. Es werden z. B. Hartschäume als Dachhimmel, Esterschäume zur Innenverkleidung der Türen sowie für ausgestanzte Sonnenblenden, Kalt- und Weichschäume für Sitzsysteme und Matratzen verwendet.

Es hat bisher nicht an Versuchen gemangelt, Antioxidantien zur Verfügung zu stellen, welche insbesondere mit Blick auf die Stabilisierung von Polymeren, welche gegen oxidativen, thermischen oder durch Licht induzierten Abbau anfällig sind, wirksam sind.

Beispielsweise können Hydroxyphenylcarbonsäureester als Antioxidantien eingesetzt werden. Zahlreiche Verbindungen aus der Klasse der Hydroxyphenylcarbonsäureester sind bekannt. Sie können beispielsweise durch Umesterung mit geeigneten Katalysatoren hergestellt werden. Solche Umesterungsverfahren sind z.B. in US-A 4716244, US-A 5481023, US-A 5563291, EP-A-1292560 beschrieben. EP-A-608089 beschreibt Ester des vorstehend genannten Strukurtyps, die Polyethylenglykolgruppen enthalten. Ihre Herstellung kann ebenfalls durch Umesterung erfolgen, wobei die bekannten stark basischen Katalysatoren wie Alkalimetalle sowie Alkalimetallamide, - alkoholate, -hydroxide und -(bi)carbonate empfohlen werden.

EP-A-2500341 beschreibt Benzofuranonderivate und deren stabilisierende Verwendung als Antioxidans in Kunststoffzusammensetzungen.

Diese und andere bekannte Antioxidantien sind zwar hilfreich, genügen aber nicht in jeder Hinsicht den hohen Erfordernissen, die ein Alterungsschutzmittel erfüllen muss, wenn es um die Stabilisierung von synthetischen Polymeren geht, insbesondere mit Blick auf Lebensdauer, Wasserabsorption, Empfindlichkeit gegen Hydrolyse, Verfahrensstabilisierung, Farbeigenschaften, Flüchtigkeit, Wanderungsverhalten, Kompatibilität und Verbesserung beim Schutz gegen Licht.

Deshalb besteht immer noch ein fortwährender Bedarf an Möglichkeiten zur Stabilisierung synthetischer Polymere, die für oxidativen, thermischen und/oder durch Licht induzierten Abbau anfällig sind.

Die konkrete Aufgabe der vorliegenden Erfindung war es vor diesem Hintergrund, Antioxidationsmittel bereitzustellen, welche zur Stabilisierung synthetischer Polymere geeignet sind, insbesondere mit Blick auf die Bereitstellung entsprechender Polyurethane, bevorzugt von Polyurethanschäumen.

In vollkommen unerwarteter Weise konnte jetzt gefunden werden, dass eine spezielle Gruppe von Verbindungen vom Typ der Hydroxyphenylcarbonsäureester dieser Zielsetzung gerecht wird.

Gegenstand der vorliegenden Erfindung ist eine Antioxidationsmittelmischung, enthaltend zumindest eine Verbindung der Formel (I) 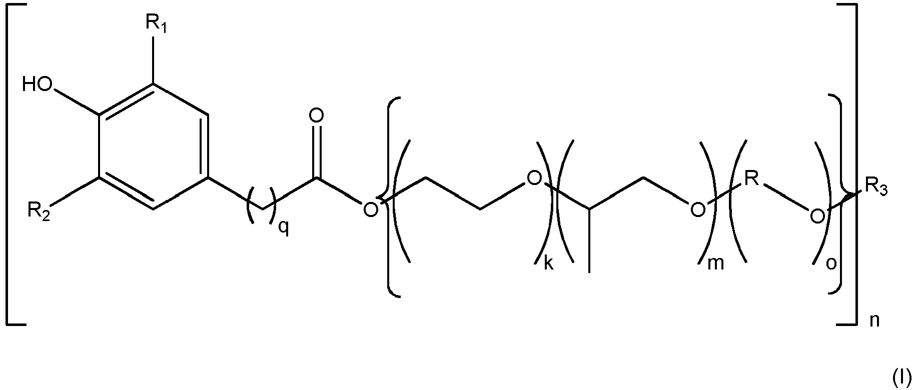 worin
- R: CH₂-CH(R^{I}), CH(R^{II})-CH(R^{II}), CH₂-C(R^{II})₂, C(R^{II})₂-C(R^{II})₂, 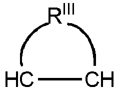 CH₂-CH-CH₂-R^{IV}, C₆H₆-CH-CH₂, oder C₆H₆-C(CH₃)-CH₂, mit
- R^{I}: C₂ bis C₂₄-Alkylrest oder Alkenrest, der linear oder verzweigt sein kann,
- R^{II}: C₂ bis C₂₄-Alkylrest oder Alkenrest, der linear oder verzweigt sein kann,
- R^{III}: C₃ bis C₆ Alkylrest, der linear angeordnet ist, und
- R^{IV}: OH, Cl, OCH₃, OCH₂-CH₃, O-CH₂-CH=CH₂, O-CH=CH₂,
Molekülrest von ein- oder mehrfach epoxidierten Fetten oder Ölen als Mono-, Di-, und Triglyceride oder Molekülrest von ein- oder mehrfach epoxidierten Fettsäuren oder deren C₁-C₂₄-Alkylestern,
- R₁ und R₂: unabhängig voneinander geradkettig oder verzweigt C₁-C₈-Alkyl, Cyclopentyl oder Cyclohexyl, insbesondere tert.-Butyl,
- q: 1, 2 oder 3, vorzugsweise 2 oder 3, insbesondere 2,
- n: eine ganze Zahl von 1 bis 30, vorzugsweise eine ganze Zahl von 1 bis 10, vorteilhafterweise 1,2,3,4,5 oder 6, z.B. 1,2,3 oder 4, insbesondere 1,
- R₃: einen n-wertigen Rest von linearem oder verzweigtem C₁-C₃₀-Alkyl, C₂-C₃₀-Alkylen, welche jeweils gegebenenfalls durch ein oder mehrere Sauerstoffatome unterbrochen sind, oder (für n = 1-12) einen n-wertigen Rest von C₅-C₁₂-Cycloalkyl, oder einen Rest R₄-[NR₅-C_{q}H_{2q}-]ₚ,
- R₄: Wasserstoff, einen n-wertigen Rest von linearem oder verzweigtem C₁-C₃₀-Alkyl, welches gegebenenfalls durch eine oder mehrere Gruppen -NR₅- unterbrochen ist, oder (für n = 1-12) einen n-wertigen Rest von C₅-C₁₂-Cycloalkyl,
- R₅: unabhängig voneinander Wasserstoff oder Methyl oder -C_{q}H_{2q}-, vorzugsweise Wasserstoff, und
- p: derjenigen Anzahl - [NR₅-C_{q}H_{2q}-]-Gruppen entspricht, welche n Reste -C_{q}H_{2q}- pro Molekül ergibt,
- k: eine ganze Zahl zwischen 0 und 50, vorzugsweise zwischen 10 und 30,
- m: eine ganze Zahl zwischen 0 und 50, z.B. 1-40, und
- o: eine ganze Zahl zwischen 0 und 50, vorzugsweise zwischen 0 und 30, insbesondere 0 darstellt,
- mit: (k + m + o) > 10,
und als weiteres Antioxidans zumindest ein Benzofuranonderivat der Formel (II) nach Maßgabe des Anspruchs 1.

Dieser Gegenstand löst die erfindungsgemäße Aufgabe. Er ermöglicht eine sehr wirkungsvolle Stabilisierung synthetischer Polymere gegen oxidativen, thermischen oder durch Licht induzierten Abbau. Als besonders wertvoll und wirkmächtig hat sich dieser Gegenstand insbesondere bei der Bereitstellung von Polyurethansystemen, vorzugsweise Polyurethanschäumen, vor allem frei gestiegenen Polyurethan-Weich(block)schäumen erwiesen. Die Erfindung ermöglicht insgesamt eine erhebliche Verbesserung des dauerhaften Erhalts der Verarbeitungs- und Leistungsmerkmale von Polyurethansystemen, insbesondere -schäumen.

Gilt in der Formel (I) k, m, o > 0 oder k, m > 0 und gleichzeitig o = 0, so ist die Reihenfolge der Monomerbausteine Ethylenoxid, Propylenoxid und (R-Oxid) in den einzelnen Polymerketten 1 bis n beliebig und k, m und o stellen Mittelwerte dar. Außerdem können die einzelnen Bausteine (EO), (PO) und (RO) entweder in Form von Blöcken, streng alternierend oder in Form von Gradienten aneinander gebunden sein. In Form von Gradienten bedeutet, dass in der Einzelkette ein Gradient der Verteilung der (EO)-, (PO)- und (RO)-Bausteine entlang der Ketten besteht.

Bei Hydroxyphenylcarbonsäureester handelt es sich um sterisch gehinderte Phenole. Aus mechanistischen Studien ist bekannt, dass die funktionelle Gruppe innerhalb der Hydroxyphenylcarbonsäureester, welche dem oxidativen Abbau von Polymeren entgegenwirkt, die sterisch gehinderte Phenoleinheit ist.

Umso überraschender ist die Tatsache, dass der Massenanteil an Phenoleinheiten in den erfindungsgemäßen Verbindungen der Formel (I) kleiner ist als in bisher bekannten Verbindungen des Typs Hydroxyphenylcarbonsäureester und dass ungeachtet dessen eine antioxidative Wirkung (insbesondere bei der Stabilisierung von synthetischen Polymeren, wie vorzugsweise Polyurethansysteme) erreicht wird, welche die Wirkung bekannter Hydroxyphenylcarbonsäureester sogar übertrifft. Es wäre eigentlich zu erwarten gewesen, dass eine größere Menge der erfindungsgemäßen Verbindungen der Formel (I) notwendig wäre, um auch nur eine den bekannten Hydroxyphenylcarbonsäureestern vergleichbare antioxidative Wirkung erzielen zu können. Das Gegenteil kann aber beobachtet werden.

Ein weiterer ganz und gar unerwarteter Vorteil der erfindungsgemäßen Verbindungen der Formel (I) liegt außerdem darin, dass der Einsatz der erfindungsgemäßen Verbindungen die Bereitstellung von Polyurethansystemen, insbesondere -schaumstoffen, ermöglicht, welche ein unerwartet gutes Emissionsverhalten aufweisen. Insofern werden die Leistungsmerkmale von Polyurethansystemen, insbesondere -schäumen sogar verbessert.

Beispielhafte Verbindungen der Formel I sind die im Folgenden abgebildeten Verbindungen Ia und Ib.

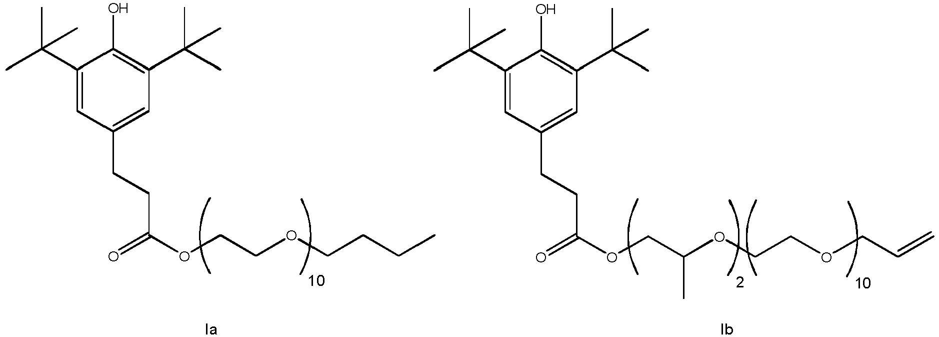

Der erfindungsgemäße Einsatz der vorgenannten Verbindungen Ia und Ib entspricht einer bevorzugten Ausführungsform der Erfindung.

Eine weitere Offenbarung im Rahmen dieser Erfindung ist ein synthetisches Polymer, vorzugsweise Polyurethansystem, insbesondere PUR-Schaum, umfassend mindestens eine Verbindung der Formel (I).

Solche Polymere, insbesondere Polyurethansysteme sind besonders alterungsunempfindlich und oxidationsresistent und insbesondere entsprechende Schäume zeigen ein unerwartet gutes Emissionsverhalten. Die Beeinflussung des Emissionsverhaltens von synthetischen Polymeren, insbesondere Polyurethan(schäum)en, ist von großer Bedeutung.

So sind z.B. bei der Herstellung und dem anschließenden Gebrauch von Polyurethansystemen, wie insbesondere -schäumen, die Freisetzung von Emissionen und Fogging problematisch. Unter Fogging versteht man die Emission von Verbindungen, die anschließend kondensieren können, wie beispielsweise im Fahrzeuginnenraum, z.B. an der Windschutzscheibe, beispielsweise unter Ausbildung eines meist trüben Belags.

Viele Verbraucher stehen deshalb Polyurethansystemen eher kritisch gegenüber und haben sogar z.T. gesundheitliche Bedenken, auch wenn objektiv keine gesundheitlichen Bedenken gerechtfertigt sind, wie Ergebnisse von Schadstoffmessungen belegen. Es besteht somit sowohl von Verbraucher- wie auch von Industrieseite ein fortwährender Wunsch nach solchen Polyurethansystemen mit geringstmöglicher Freisetzung von Emissionen.

Im Rahmen dieser Erfindung konnte nun gefunden werden, dass die erfindungsgemäßen Verbindungen der Formel (I) die Bereitstellung von Polyurethansystemen, insbesondere PUR-Schaum, mit geringstmöglicher Freisetzung von Emissionen und geringstmöglichem Fogging ermöglichen, verglichen mit Polyurethansystemen, bei welchen herkömmliche Antioxidantien eingesetzt worden sind.

Als eine etablierte Prüfmethode zur Beurteilung der Emissionen hat sich im Markt beispielsweise die DaimlerChrysler Prüfanweisung VDA 278: "Thermodesorptionsanalyse organischer Emissionen zur Charakterisierung nichtmetallischer KFZ-Werkstoffe" vom Oktober 2011 bewährt. Der Wert für die Emissionen flüchtiger Verbindungen wird im Folgenden als VOC-Wert (VOC-Wert = Volatile Organic Compounds) bezeichnet. Der Wert für die Emissionen kondensierbarer Verbindungen, das entspricht dem Fogging, werden im Folgenden als Fog-Wert bezeichnet. Geeignete Methoden zur Bestimmung von VOC und Fogging sind im Beispielteil genau beschrieben.

Der Einsatz der erfindungsgemäßen Verbindungen der Formel (I) ermöglicht vorteilhafterweise die Herstellung von Polyurethansystemen, insbesondere -schäumen, die sehr geringe Werte von flüchtigen organischen (VOC) und kondensierbaren Verbindungen (Fogging) aufweisen. Es konnten Werte für VOC von < 100 ppm und für Fogging von < 250 ppm erreicht werden. Die Werte für VOC und Fogging können insbesondere mittels Thermodesorptionsanalyse bestimmt werden. Der Einsatz der erfindungsgemäßen Verbindungen der Formel (I) ermöglicht darüber hinaus vorteilhafterweise die Herstellung von Polyurethansystemen, insbesondere -schäumen, die besonders geruchsarm sind. Der Einsatz der erfindungsgemäßen Verbindungen der Formel (I) ermöglicht weiterhin vorteilhafterweise die Herstellung von Polyurethansystemen, insbesondere - schäumen, die besonders alterungsbeständig sind. Der Einsatz der Verbindungen der Formel (I) hat außerdem den Vorteil, dass diese komplikationslos in bestehenden Produktionsprozessen und Produktionsanlagen genutzt werden können.

Die erfindungsgemäßen Verbindungen der Formel (I) können durch jedes geeignete Verfahren, das auf dem Gebiet der Herstellung von Verbindungen dieses Typs allgemein bekannt ist, hergestellt werden, einschließlich verschiedenen Veresterungsverfahren. Drei Beispiele für solche Verfahren sind in den nachfolgenden Reaktionsschemata exemplarisch erläutert und jeweils als Verfahren A, Verfahren B und Verfahren C bezeichnet.

### 1) Verfahren A

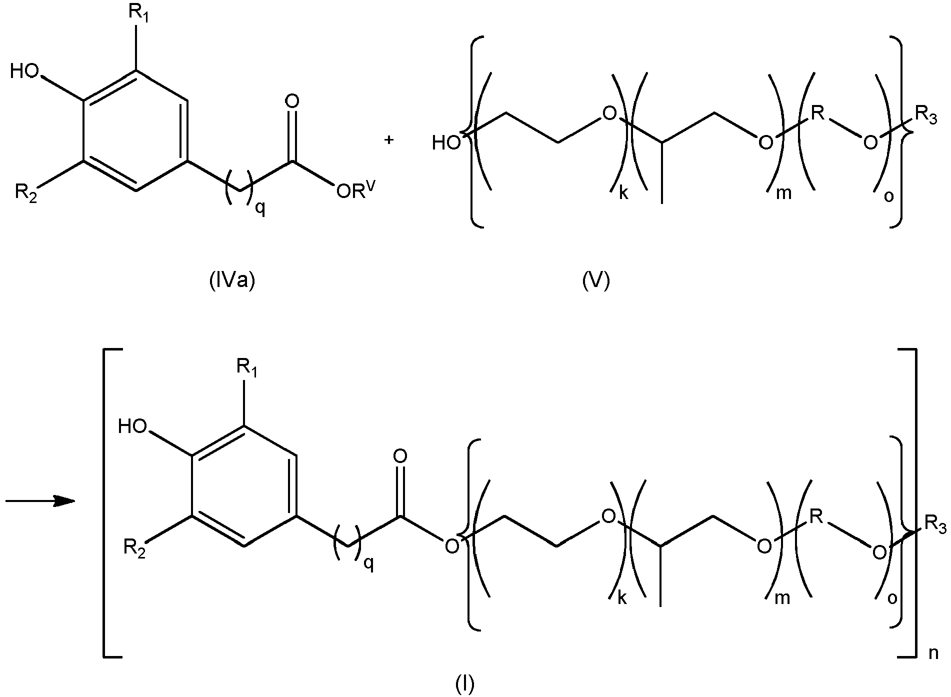

### 2) Verfahren B

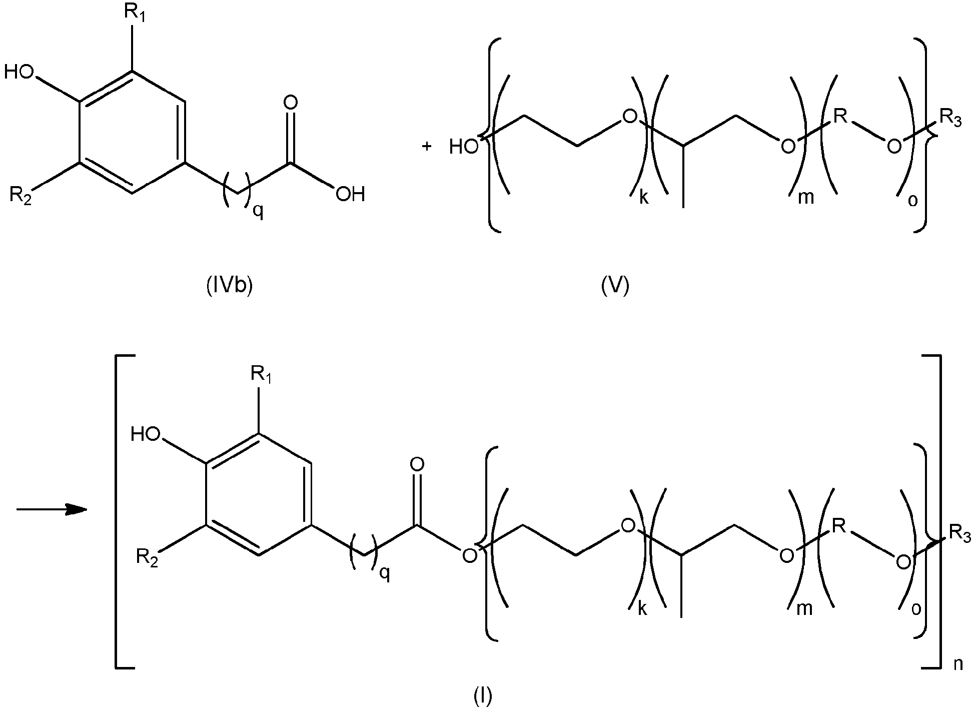

### 3) Verfahren C

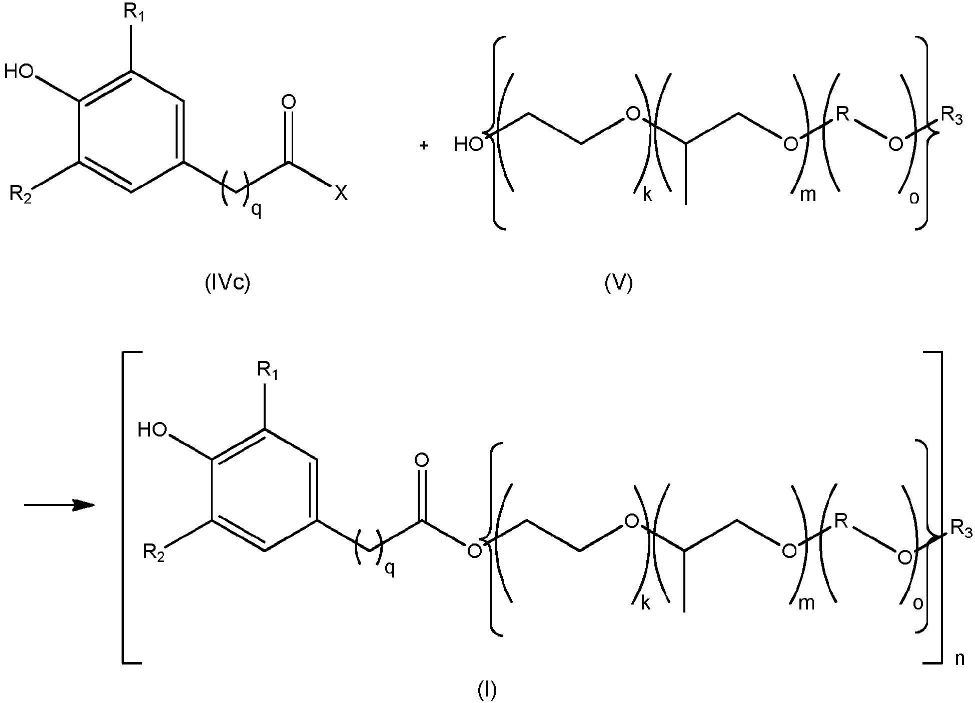

In den obigen Reaktionsschemata haben R, R₁, R₂, R₃ k, n, q, m und o die oben für Formel (I) angegebene Bedeutung, bedeutet R^{V} eine geradkettig oder verzweigtkettige Alkylgruppe mit 1 bis 4 Kohlenstoffatomen, wie beispielsweise eine Methyl-, Ethyl-, Propyl-, Isopropyl-, Butyl- oder Isobutylgruppe, vorzugsweise eine Methylgruppe, und bedeutet X ein Halogenatom wie ein Chlor- oder Bromatom.

Die Ausgangsmaterialien der Formel (V) sind Addukte von Alkylenoxiden mit verschiedenen Alkoholen, die als Alkylenglycolalkylmonoether oder als O-alkylierte Alkylenglycole beschrieben werden können. Es ist zu bedenken, dass im Fall von Polyalkylenoxid-Addukten die im Handel erhältlichen Produkte oftmals Gemische aus mehreren Einzelverbindungen mit unterschiedlichen Anzahlen von Ethylenoxid-, Propylenoxid- und/oder R-Oxid-Einheiten sind. Falls ein solches Gemisch als Ausgangsmaterial verwendet wird, besteht das Endprodukt der Formel (I) aus einem entsprechenden Gemisch. Deshalb versteht sich, dass bei solchen Gemischen die Indices k, m und o eine Durchschnittsanzahl von Ethylenoxid-, Propylenoxid bzw. R-Oxid-Einheiten bedeuten können, so dass sie für das Gesamtgemisch Bruchzahlen sein können.

Verfahren A umfasst eine Umesterung zwischen dem Alkylenglycolmonoether der Formel (V) und dem substituierten Phenylpropionsäureester der Formel (IVa). Diese Umsetzung kann nach Wunsch in Gegenwart oder Abwesenheit eines Lösungsmittels und in Gegenwart eines Umesterungskatalysators durchgeführt werden.

Wenn bei dieser Umsetzung ein Lösungsmittel verwendet wird, umfassen geeignete inerte Lösungsmittel beispielsweise Ether wie Diisopropylether, Dioxan und Tetrahydrofuran, halogenierte Kohlenwasserstoffe wie Tetrachlorkohlenstoff und Dichlorethan, lineare oder cyclische aliphatische Kohlenwasserstoffe wie Hexan, Heptan, Oktan, Isooktan, Cyclohexan, Methylcyclohexan, Ethylcyclohexan und Kerosin, und aromatische Kohlenwasserstoffe wie Benzol, Toluol und Xylol. Aromatische Kohlenwasserstoffe sind bevorzugt.

Geeignete Umesterungskatalysatoren umfassen beispielsweise Alkalimetalle wie Lithium, Natrium und Kalium, Alkalimetallhydride wie Lithiumhydrid, Alkalimetallamide wie Lithiumamid, Natriumamid und Lithium-N,N-diisopropylamid, Alkalimetallalkoxide wie Natriummethoxid, Natriumethoxid und Kalium-t-butoxid, Alkalimetallhydroxide wie Lithiumhydroxid, Natriumhydroxid und Kaliumhydroxid, Alkalimetallcarbonate wie Lithiumcarbonat, Natriumcarbonat und Kaliumcarbonat, Alkalimetallbicarbonate wie Lithiumbicarbonat, Natriumbicarbonat und Kaliumbicarbonat, Carbonsäuresalze von Alkali- und Erdalkalimetallen (beispielsweise Acetate oder Formiate) wie Lithiumacetat, Natriumacetat, Kaliumacetat oder Magnesiumacetat und Lithuiumformiat, Natriumformiat oder Kaliumformiat, Aluminiumalkoholate und -phenolate, Titan(IV)alkoxide wie Titan(IV)tetraisopropoxid und Titan(IV)tetrabutoxid, Metalloxide wie Zinnoxid, metallorganische Zinn(IV)verbindungen wie Dibutylzinnoxid oder organische Säuren wie Benzolsulfonsäure, p-Toluolsulfonsäure, Trifluormethansulfonsäure und Methansulfonsäure, und Mineralsäuren wie Chlorwasserstoffsäure und Schwefelsäure, wobei Mineralsäuren und Sulfonsäuren, insbesondere Schwefelsäure als Mineralsäure und p-Toluolsulfonsäure als Sulfonsäure, bevorzugt sind. Die Alkalimetallalkoxide sind bevorzugt.

Reaktionstemperatur und -zeit können in Abhängigkeit von den Ausgangsmaterialien, dem Katalysator und dem (gegebenenfalls verwendeten) Lösungsmittel variieren. Jedoch beträgt die Temperatur im Allgemeinen 50 bis 200°C, stärker bevorzugt 80 bis 140°C, während die Reaktionszeit gewöhnlich 2 bis 24 Stunden, stärker bevorzugt 4 bis 12 Stunden, beträgt.

Nach Beendigung der Umesterungsreaktion kann das gewünschte Produkt der Formel (I) durch herkömmliche Techniken abgetrennt werden. Beispielsweise wird bei basischer Reaktionsführung das Reaktionsgemisch mit einer verdünnten Mineralsäure (z.B. verdünnter Chlorwasserstoffsäure oder Schwefelsäure) gewaschen und neutralisiert, wonach unlösliche Bestandteile entfernt werden (z.B. durch Filtration) und die erhaltene Flüssigkeit über einem Dehydratisierungsmittel (z.B. wasserfreiem Magnesiumsulfat) getrocknet und das Lösungsmittel verdampft wird. Falls erwünscht, kann das erhaltene Produkt gereinigt werden, beispielsweise durch Destillation unter vermindertem Druck oder durch Säulenchromatographie.

Verfahren B umfasst die Veresterung des Alkylenglycolmonoethers der Formel (V) mit der substituierten Phenylpropionsäure der Formel (IVb). Diese Umsetzung wird vorzugsweise in einem inerten Lösungsmittel und in Gegenwart eines Säurekatalysators durchgeführt.

Geeignete inerte Lösungsmittel, die bei dieser Umsetzung verwendet werden können, umfassen beispielsweise Ether wie Diisopropylether, Dioxan und Tetrahydrofuran, halogenierte Kohlenwasserstoffe wie Methylenchlorid, Tetrachlorkohlenstoff und Dichlorethan, aliphatische Kohlenwasserstoffe wie Hexan, Heptan, Oktan, Ethylcyclohexan und Kerosin, und aromatische Kohlenwasserstoffe wie Benzol, Toluol und Xylol. Aromatische Kohlenwasserstoffe sind bevorzugt.

Die Säurekatalysatoren, die bei dieser Reaktion verwendet werden können, umfassen beispielsweise Sulfonsäuren wie Benzolsulfonsäure, p-Toluolsulfonsäure, Trifluormethansulfonsäure und Methansulfonsäure, und Mineralsäuren wie Chlorwasserstoffsäure und Schwefelsäure, wobei Mineralsäuren und Sulfonsäuren, insbesondere Schwefelsäure als Mineralsäure und p-Toluolsulfonsäure als Sulfonsäure, bevorzugt sind.

Reaktionstemperatur und -zeit können in Abhängigkeit von den Ausgangsmaterialien, dem Lösungsmittel und dem Katalysator variieren, jedoch beträgt die Temperatur im Allgemeinen 60 bis 200°C, stärker bevorzugt 100 bis 150°C, und die Reaktionszeit im Allgemeinen 3 bis 24 Stunden, stärker bevorzugt 4 bis 12 Stunden.

Nach Beendigung der Veresterungsreaktion kann das gewünschte Produkt der Formel (I) durch herkömmliche Techniken abgetrennt werden. Beispielsweise wird das Reaktionsgemisch mit einer wässrigen Alkalilösung (z.B. wässrigem Natriumbicarbonat) gewaschen und neutralisiert, wonach unlösliche Bestandteile entfernt werden (z.B. durch Filtration) und die erhaltene Flüssigkeit über einem Dehydratisierungsmittel (z.B. wasserfreiem Magnesiumsulfat) getrocknet und das Lösungsmittel verdampft wird, so dass das Produkt der Formel (I) erhalten wird. Falls erwünscht, kann das Produkt gereinigt werden, beispielsweise durch Destillation unter vermindertem Druck oder durch Säulenchromatographie.

Verfahren C umfasst die Veresterung des Alkylenglycolmonoethers der Formel (V) mit dem substituierten Phenylpropionsäurehalogenid der Formel (IVc). Diese Reaktion wird vorzugsweise in einem inerten Lösungsmittel und in Gegenwart eines Halogenwasserstoff-Fängers durchgeführt.

Beispiele für bei dieser Reaktion geeignete Lösungsmittel umfassen die bereits für die Reaktion des Verfahrens A aufgelisteten.

Beispiele für geeignete Halogenwasserstoff-Fänger umfassen Alkalimetallhydroxide wie Lithiumhydroxid, Natriumhydroxid und Kaliumhydroxid, Alkalimetallcarbonate wie Lithiumcarbonat, Natriumcarbonat und Kaliumcarbonat, Alkalimetallbicarbonate wie Lithiumbicarbonat, Natriumbicarbonat und Kaliumbicarbonat, aliphatische tertiäre Amine wie Triethylamin, Trioctylamin, N- Methylmorpholin und N,N-Dimethylpiperazin, und Pyridine wie Pyridin und N,N-Dimethylaminopyridin. Triethylamin und die Pyridine sind bevorzugt.

Reaktionstemperatur und -zeit können in Abhängigkeit von den Ausgangsmaterialien, dem Lösungsmittel und Halogenwasserstoff- Fänger, die verwendet werden, variieren. Jedoch beträgt die Reaktionstemperatur gewöhnlich 0 bis 120°C, stärker bevorzugt 10 bis 60°C, während die Reaktionszeit gewöhnlich 1 Stunde bis 12 Stunden, stärker bevorzugt 4 bis 8 Stunden, beträgt.

Nach Beendigung der Umsetzung kann das gewünschte Produkt der Formel (I) durch herkömmliche Techniken abgetrennt werden. Beispielsweise wird das Reaktionsgemisch mit einer verdünnten Mineralsäure (z.B. verdünnter Chlorwasserstoffsäure oder Schwefelsäure) gewaschen, wonach unlösliche Bestandteile entfernt werden (z.B. durch Filtration) und die erhaltene Flüssigkeit über einem Dehydratisierungsmittel (z.B. wasserfreiem Magnesiumsulfat) getrocknet und das Lösungsmittel verdampft wird, wodurch das gewünschte Produkt erhalten wird. Falls erwünscht, kann das Produkt gereinigt werden, beispielsweise durch Destillation unter vermindertem Druck oder Säulenchromatographie.

Die erfindungsgemäßen Verbindungen der Formel (I) ermöglichen weiterhin die Bereitstellung von Antioxidationsmittelmischungen. Eine Antioxidationsmittelmischung im Sinne dieser Erfindung enthält neben der Verbindung der Formel (I) zumindest ein weiteres Antioxidans, nämlich zumindest ein Benzofuranonderivat der Formel (II) nach Maßgabe des Anspruchs 1. Daneben können gewünschtenfalls noch weitere Inhaltsstoffe enthalten sein, wie z.B. Lösungsmittel usw. Diese Lösungsmittel sind vorzugsweise ausgewählt aus Wasser, Alkoholen, insbesondere Polyethermonoolen oder -polyolen, vorzugsweise bestehend aus H-funktionellen Startersubstanzen, an welche mittels Alkoxylierung Alkylenoxide (Epoxide) mit 2-24 Kohlenstoffatomen, vorzugsweise Ethylenoxid und/oder Propylenoxid, angelagert wurden und die ein Molekulargewicht von vorzugsweise 200 - 8000 g/mol, bevorzugt von 300 - 5000 g/mol, besonders bevorzugt von 500 - 1000 g/mol, und einen PO-Gehalt von vorzugsweise 10 - 100 Gew.%, bevorzugt von 50 - 100 Gew.% aufweisen sowie Polyestermonolen oder -polyolen mit einem Molekulargewicht vorzugsweise im Bereich von 200 bis 4500 g/mol, Glykolen, Alkoxylaten, Carbonaten, Ether, Ester, verzweigten oder linearen aliphatischen oder aromatischen Kohlenwasserstoffen und/oder Ölen synthetischer und/oder natürlicher Herkunft.

Überraschenderweise konnte im Rahmen dieser Erfindung allerdings gefunden werden, dass ausgerechnet bestimmte Benzofuranonderivate der unten genannten Formel (II) die Wirkmächtigkeit der Verbindungen der Formel (I) ganz besonders stark unterstützen, so dass von einem synergistischen Zusammenwirken gesprochen werden kann.

Demgemäß eingesetzte Verbindungen des Benzofuran-2-on-Typs im Sinne dieser Erfindung sind Verbindungen der Formel (II). 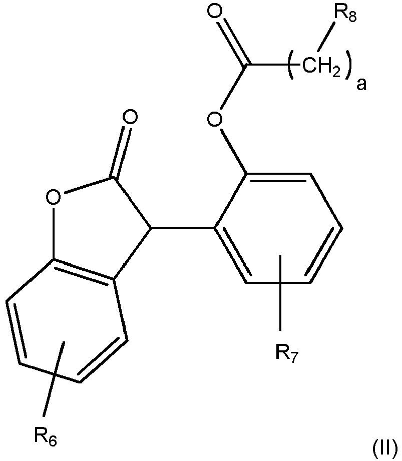 worin
- a: einer ganzen Zahl zwischen 0 und 7, vorzugsweise 0 - 3, z.B.1 oder 2,
- R₆ und R₇: unabhängig voneinander H oder C₁-C₈-Alkyl, z.B. tert.-Butyl,
- R₈: H oder einem aromatischen Rest 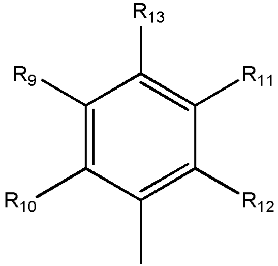 mit
- R₉ und R₁₀: unabhängig voneinander H oder C₁-C₆-Alkyl, wobei nicht beide eine C₁-C₆-Alkyl darstellen,
- R₁₁ und R₁₂: unabhängig voneinander H oder C₁-C₆-Alkyl, wobei nicht beide eine C₁-C₆-Alkyl darstellen,
- R₁₃: H oder OH, insbesondere OH
entspricht.

Benzofuran-2-on-Stabilisatoren der Formel (II) sind in der Literatur als solche bekannt. Insbesondere sei hierbei auf EP 2500341 verwiesen.

Insbesondere kann im Sinne dieser Erfindung als Verbindung des Benzofuran-2-on-Typs die Verbindung (IIa) eingesetzt werden,
4-Tert-butyl-2-(5-tert-butyl-2-oxo-2,3-dihydro-1-benzofuran-3-yl)phenyl-3,5-ditertbutyl-4-hydroxybenzoat (IIa)

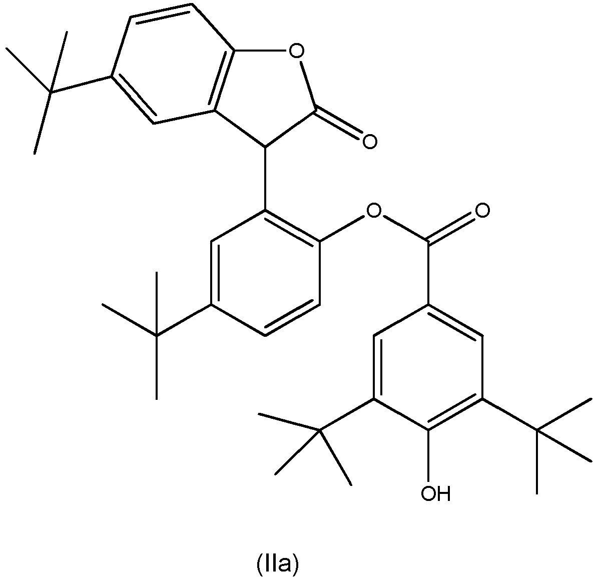

Der Einsatz dieser Verbindung der Formel (IIa) zeigte besonders vorteilhafte Ergebnisse mit Blick auf die angestrebten Wirkungen.

Gemäß einer bevorzugten Ausführungsform der Erfindung enthält eine erfindungsgemäße Antioxidationsmittelmischung Verbindung(en) der Formel (I) in einer Menge von 75 bis 99 Gew.-%, vorzugsweise 80 bis 98 Gew.-%, insbesondere 90 bis 95 Gew.-% und Verbindung(en) der Formel (II) in einer Menge von 1 bis 25 Gew.-%, vorzugsweise 2 bis 20 Gew.-%, insbesondere 5 bis 10 Gew.-%, Gew.-% bezogen auf das Gesamtgewicht der eingesetzten Verbindungen der Formeln (I) und (II).

Weiterhin konnte gefunden werden, dass erfindungsgemäße Antioxidationsmittelmischungen, die sowohl Verbindung(en) der Formel (I) als auch (II) enthalten, insbesondere in Kombination mit einem Phosphit (Ester der Phosphorigsäure), besonders vorteilhaft sind. Demgemäß bevorzugt einsetzbare Phosphite sind solche mit der allgemeinen Formel (III) 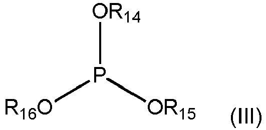 wobei
R₁₄, R₁₅ und R₁₆ unabhängig voneinander einen aromatischen oder aliphatischen, linearen oder verzweigten Rest von C₁-C₃₀-Alkyl oder C₂-C₃₀-Alkylen, welche jeweils gegebenenfalls durch ein oder mehrere Sauerstoffatome unterbrochen sind, darstellen.

Besonders bevorzugte Beispiele für solche Phosphite sind die kommerziell erhältlichen Verbindungen (IIIa), (IIIb) und (IIIc).

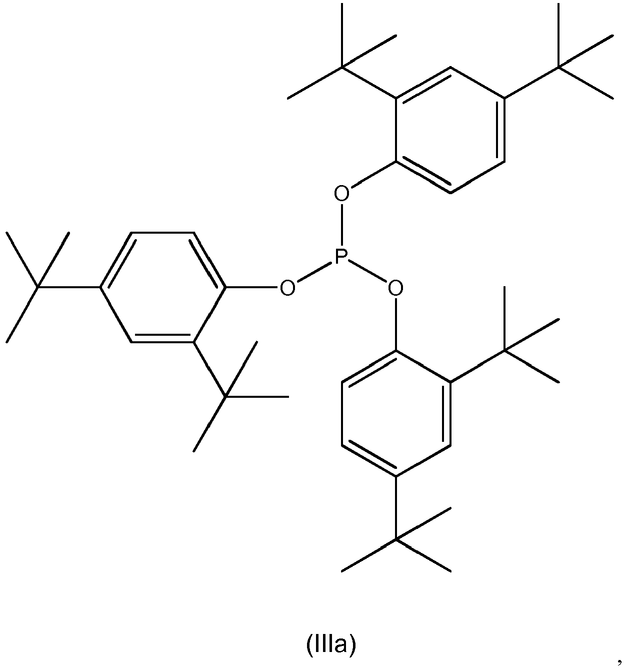

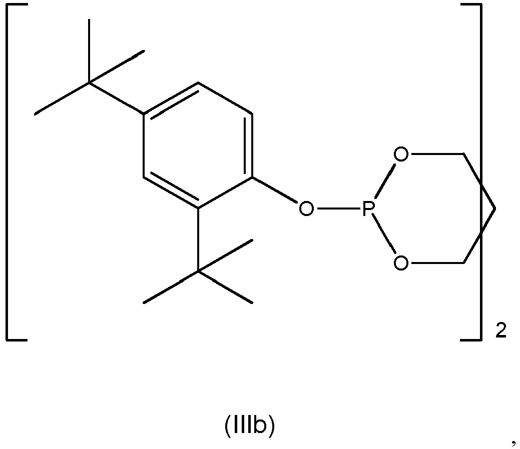

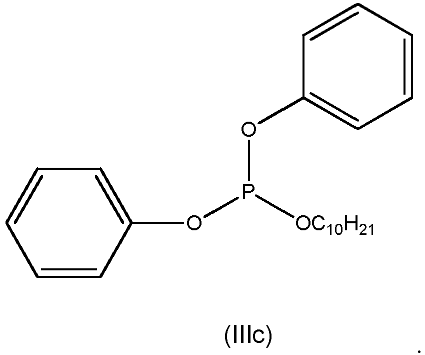

Insbesondere kann im Sinne dieser Erfindung als Verbindung des Phosphit-Typs die Verbindung (IIIa) eingesetzt werden, Tris(2,4-di-tert-butylphenyl)phosphit (IIIa).

Der Einsatz dieser Verbindung der Formel (IIIa) zeigte ganz besonders vorteilhafte Ergebnisse mit Blick auf die angestrebten Wirkungen.

Gemäß einer ganz besonders bevorzugten Ausführungsform der Erfindung enthält eine erfindungsgemäße Antioxidationsmittelmischung Verbindung(en) der Formel (I) in einer Menge von 75 bis 99 Gew.-%, vorzugsweise 80 bis 98 Gew.-%, insbesondere 90 bis 95 Gew.-% und Verbindung(en) der Formel (II) in einer Menge von 1 bis 25 Gew.-%, vorzugsweise 2 bis 20 Gew.-%, insbesondere 5 bis 10 Gew.-% und Verbindungen der Formel (III) in einer Menge von 0,1 bis 20 Gew.-%, vorzugsweise 0,2 bis 15 Gew.-%, insbesondere 0,5 bis 10 Gew.-%, Gew.-% bezogen auf das Gesamtgewicht der eingesetzten Verbindungen der Formeln (I), (II) und (III).

Ein weiterer Gegenstand dieser Erfindung ist vor dem geschilderten Hintergrund ein Verfahren zur Herstellung von Polyurethan-Systemen, insbesondere PUR-Schaum, durch Umsetzung mindestens einer Polyolkomponente mit mindestens einer Isocyanatkomponente in Gegenwart eines oder mehrerer Katalysatoren, die die Reaktionen Isocyanat-Polyol und/oder Isocyanat-Wasser und/oder die Isocyanat-Trimerisierung katalysieren, wobei die Umsetzung in Gegenwart einer Antioxidationsmittelmischung, wie zuvor beschrieben, durchgeführt wird.

Vorzugsweise wird das PUR-System, insbesondere der PUR-Schaum, dadurch erzeugt, dass eine Mischung enthaltend zumindest einen Urethan- und/oder Isocyanurat-Katalysator, zumindest ein Treibmittel und/oder Wasser, zumindest eine Isocyanatkomponente und eine Polyolkomponente in Gegenwart einer Antioxidationsmittelmischung, wie zuvor beschrieben, verschäumt wird.

Neben den genannten Komponenten kann die Mischung weitere Bestandteile aufweisen, wie z. B. optional (weitere) Treibmittel, optional Präpolymere, optional Flammschutzmittel und optional weitere Additive (die von den in der erfindungsgemäßen Additivzusammensetzung genannten verschieden sind), wie z. B. Füllstoffe, Emulgatoren, die auf der Umsetzung von hydroxyfunktionellen Verbindungen mit Isocyanat beruhen, Schaumstabilisatoren, wie z. B. Si-haltige und nicht Si-haltige, insbesondere Si-haltige und nicht Si-haltige organische Stabilisatoren und Tenside, Viskositätssenker, Farbstoffe, UV-Stabilisatoren oder Antistatika. Es versteht sich von selbst, dass der Fachmann zur Herstellung der unterschiedlichen Polyurethanweichschaumstoff-Typen, d.h. Heiß-, Kalt- oder Ester-Polyurethanweichschaumstoffe, die hierfür jeweils notwendigen Substanzen, wie z. B. Isocyanat, Polyol, Präpolymer, etc. entsprechend auswählt, um den jeweils gewünschten Polyurethanweichschaumstoff-Typ zu erhalten.

Nachstehend sind eine Reihe von Schutzrechten angegeben, die geeignete Komponenten und Verfahren zur Herstellung der unterschiedlichen Polyurethanweichschaumstoff-Typen, d.h. Heiß-, Kalt- sowie Ester-Polyurethanweichschaumstoffe beschreiben, auf die im vollen Umfang Bezug genommen wird: EP 0152878 A1, EP 0 409 035 A2, DE 102005050473 A1, DE 19629161 A1, DE 3508292 A1, DE 4444898 A1, EP 1061095 A1, EP 0532939 B1, EP 0867464 B1, EP 1683831 A1 und DE 102007046860 A1.

Weitere Angaben zu verwendbaren Ausgangsstoffen, Katalysatoren sowie Hilfs- und Zusatzstoffen finden sich beispielsweise im Kunststoff-Handbuch, Band 7, Polyurethane, Carl-Hanser-Verlag München, 1. Auflage 1966, 2. Auflage, 1983 und 3. Auflage, 1993.

Die nachstehenden Verbindungen, Komponenten und Additive sind lediglich beispielhaft genannt und können durch andere dem Fachmann bekannte Stoffe ersetzt werden.

Tenside, die bei der Herstellung von Polyurethanweichschaumstoffen eingesetzt werden können, können z. B. ausgewählt sein aus der Gruppe umfassend nichtionische Tenside und/oder amphotere Tenside.

Als Tenside können erfindungsgemäß beispielsweise auch polymere Emulgatoren, wie Polyalkylpolyoxyalkylpolyacrylate, Polyvinylpyrrolidone oder Polyvinylacetate verwendet werden. Ebenso können als Tenside/Emulgatoren Präpolymere, die durch Umsetzung von geringen Mengen von Isocyanaten mit Polyolen erhalten werden (sog. Oligourethane), und die vorzugsweise gelöst in Polyolen vorliegen, eingesetzt werden.

Als Schaumstabilisatoren können vorzugsweise jene eingesetzt werden, die aus dem Stand der Technik bekannt sind und auf die auch üblicherweise zur Polyurethanschaumstabilisierung zurückgegriffen wird. Hierbei kann es sich sowohl um Si-haltige und nicht Si-haltige, insbesondere Si-haltige und nicht Si-haltige organische Stabilisatoren und Tenside handeln. Die Si-haltigen Stabilisatoren werden weiterhin darin unterschieden, ob der Polyoxyalkylenblock mit dem Polysiloxanblock durch eine hydrolytisch stabile C-Si-Bindung (wie beispielsweise in EP 2 182 020) oder die hydrolytisch weniger stabile C-O-Si-Bindung verknüpft ist. Die zur Polyurethanschaumstabilisierung verwendbaren SiC-Polysiloxan-Polyoxyalkylen-Blockmischpolymerisate können z.B. durch edelmetallkatalysierte Hydrosilylierung von ungesättigten Polyoxyalkylenen mit SiH-funktionellen Siloxanen, sogenannten Wasserstoffsiloxanen, wie beispielsweise in der EP 1 520 870 beschrieben, hergestellt werden. Die Hydrosilylierung kann batchweise oder kontinuierlich, wie beispielsweise in der DE 198 59 759 C1 beschrieben, durchgeführt werden.

Eine Vielzahl weiterer Schriften, wie beispielsweise die EP 0493836 A1, US5565194 oder EP 1350804 offenbaren jeweils speziell zusammengesetzte Polysiloxan-Polyoxyalkylen-Blockmischpolymerisate zur Erfüllung spezifischer Anforderungsprofile für Schaumstabilisatoren in diversen Polyurethanschaumformulierungen.

Als Biozide können handelsübliche Produkte verwendet werden, wie Chlorophen, Benzisothiazolinon, Hexahydro-1,3,5-tris(hydroxyethyl-s-triazin), Chlormethylisothiazolinon, Methylisothiazolinon oder 1,6-Dihydroxy-2,5-dioxohexan, die unter den Handelsnamen BIT 10, Nipacide BCP, Acticide MBS, Nipacide BK, Nipacide Cl, Nipacide FC bekannt sind.

Geeignete Flammschutzmittel im Sinne dieser Erfindung sind alle Substanzen, die im Stand der Technik als dafür geeignet betrachten werden. Bevorzugte Flammschutzmittel sind beispielsweise flüssige organische Phosphor-Verbindungen, wie halogenfreie organische Phosphate, z.B. Triethylphosphat (TEP), halogenierte Phosphate, z.B. Tris(1-chlor-2-propyl)phosphat (TCPP), Tris(1,3-dichlor-2-propyl)phosphat (TDCPP) und Tris(2-chlorethyl)phosphat (TCEP) und organische Phosphonate, z.B. Dimethylmethanphosphonat (DMMP), Dimethylpropanphosphonat (DMPP), oder Feststoffe wie Ammoniumpolyphosphat (APP) und roter Phosphor. Des Weiteren sind als Flammschutzmittel halogenierte Verbindungen, beispielsweise halogenierte Polyole, sowie Feststoffe wie Blähgraphit und Melamin geeignet. Alle diese Flammschutzmittel und Kombinationen daraus können im Sinne dieser Erfindung vorteilhafterweise benutzt werden, darin enthalten auch alle kommerziell erhältlichen Flammschutzmittel der Firmen Great Lakes Solutions (Chemtura) (z.B.: DP-54™, Firemaster® BZ-54 HP, Firemaster® 550, Firemaster® 552, Firemaster® 600, Firemaster® 602, Reofos® 50, Reofos® 65, Reofos® 95, Kronitex® CDP), ICL Industrial Products (z.B.: FR-513, FR-1210, FR-1410, Fyrol™ FR-2, Fyrol™ 38, Fyrol™ HF-5, Fyrol™ A300TB, Fyrol™ PCF, Fyrol™ PNX, Fyrol™ PNX-LE), Clariant (z.B.: Exolit® OP 550 oder Exolit® OP 560).

Oftmals werden alle Komponenten außer den Polyolen und Isocyanaten vor dem Verschäumen zu einer sogenannten Aktivatorlösung vermischt. Diese enthält dann vorzugsweise u.a. die erfindungsgemäß verwendbare Additivzusammensetzung, also Verbindungen der Formel (I) oder erfindungsgemäße Antioxidationsmittelmischung, Schaumstabilisatoren, Katalysatoren bzw. Katalysatorkombination, das Treibmittel, beispielsweise Wasser, sowie eventuell weitere Additive, wie Flammschutz, Farbe, Biozide etc., je nach Rezeptur des Polyurethanweichschaumstoffs. Auch eine solche Aktivatorlösung kann eine erfindungsgemäße Zusammensetzung sein.

Bei den Treibmitteln unterscheidet man zwischen chemischen und physikalischen Treibmitteln. Zu den chemischen Treibmitteln gehört z. B. Wasser, dessen Reaktion mit den Isocyanatgruppen zur Bildung von CO₂ führt. Die Rohdichte des Schaumstoffes lässt sich durch die zugegebene Wassermenge steuern, wobei die bevorzugten Einsatzmengen an Wasser z.B. zwischen 0,5 und 10 Teilen, bevorzugt zwischen 1 und 7 Teilen, besonders bevorzugt zwischen 1 und 5 Teilen, bezogen auf 100,0 Teile Polyol, liegen. Darüber hinaus können alternativ und/oder auch zusätzlich, physikalische Treibmittel, eingesetzt werden. Dabei handelt es sich um Flüssigkeiten, welche gegenüber den Rezepturbestandteilen inert sind und Siedepunkte unter 100 °C, vorzugsweise unter 50 °C, insbesondere zwischen -50 °C und 30 °C bei Atmosphärendruck aufweisen, so dass sie unter dem Einfluss der exothermen Polyadditionsreaktion verdampfen. Beispiele derartiger, vorzugsweise verwendbarer Flüssigkeiten sind Ketone wie Aceton und/oder Methylethylketon, Kohlenwasserstoffe, wie n-, iso- oder Cyclopentan, n- oder iso-Butan und Propan, Cyclohexan, Ether, wie Dimethylether und Diethylether, halogenierte Kohlenwasserstoffe, wie Methylenchlorid, Tetrafluorethan, Pentafluorpropan, Heptafluorpropan, Pentafluorbutan, Hexafluorbutan und/oder Dichlormonofluorethan, Trichlorfluormethan, Dichlortetrafluorethan und 1,1,2-Trichlor-1,2,2-trifluorethan. Des Weiteren kann auch Kohlendioxid eingesetzt werden. Auch Gemische dieser niedrigsiedenden Flüssigkeiten untereinander und/oder mit anderen substituierten oder unsubstituierten Kohlenwasserstoffen können verwendet werden. Die Verschäumung kann sowohl unter Normaldruck, als auch unter vermindertem Druck ablaufen (VPF-Technologie).

Die Menge des physikalischen Treibmittels liegt dabei vorzugsweise im Bereich zwischen 1 und 120 Gew.-Teilen, insbesondere zwischen 1 und 15 Gew.-Teilen, die Menge an Wasser vorzugsweise im Bereich zwischen 0,5 bis 10 Gew.-Teilen, insbesondere 1 bis 5 Gew.-Teilen, jeweils bezogen auf 100 Gew.-Teile Polyol. Kohlendioxid wird von den physikalischen Treibmitteln bevorzugt, welches bevorzugt in Kombination mit Wasser als chemischem Treibmittel verwendet wird.

Die erfindungsgemäße Aktivatorlösung kann zusätzlich alle üblichen im Stand der Technik für Aktivatorlösungen bekannten Zusätze enthalten. Die Zusätze können ausgewählt sein aus der Gruppe umfassend Flammschutzmittel, UV-Stabilisatoren, Farbstoffe, Biozide, Pigmente, Zellöffner, Vernetzer und dergleichen.

Zur Herstellung eines erfindungsgemäßen PUR-Schaums, insbesondere Polyurethanweichschaumstoffs, wird vorzugsweise ein Gemisch (Mischung) aus Polyol, di- oder polyfunktionellem Isocyanat, erfindungsgemäßem Additiv, also Verbindungen der Formel (I) oder erfindungsgemäße Antioxidationsmittelmischung, Aminkatalysator, Kalium-, Zink- und/oder Zinnorganischer-Verbindung oder andere metallhaltige Katalysatoren, Schaumstabilisator, Treibmittel, vorzugsweise Wasser zur Bildung von CO₂ und, falls nötig, Zusatz von physikalischen Treibmitteln, gegebenenfalls unter Zugabe von Flammschutzmitteln, UV-Stabilisatoren, Farbpasten, Bioziden, Füllstoffen, Vernetzern oder sonstigen üblichen Verarbeitungshilfsmitteln, umgesetzt. Ein solches Gemisch stellt ebenfalls einen Gegenstand der Erfindung dar. Ein Gemisch, umfassend das erfindungsgemäß einzusetzende Additiv, also Verbindungen der Formel (I) oder erfindungsgemäße Antioxidationsmittelmischung, und Polyol, stellt ebenfalls einen Gegenstand der Erfindung dar sein.

Als Isocyanate können organische Isocyanatverbindungen verwendet werden, die mindestens zwei Isocyanat-Gruppen enthalten. Generell kommen die an sich bekannten aliphatischen, cycloaliphatischen, araliphatischen und vorzugsweise aromatischen mehrwertigen Isocyanate in Frage. Besonders bevorzugt werden Isocyanate in einem Bereich von 60 bis 140 mol % relativ zu der Summe der isocyanatverbrauchenden Komponenten eingesetzt.

Im Einzelnen seien beispielhaft genannt: Alkylendiisocyanate mit 4 bis 12 Kohlenstoffatomen im Alkylenrest, wie 1,12-Dodecandiisocyanat, 2-Ethyltetramethylendiisocyanat-1,4, 2-Methylpentamethylendiisocyanat-1,5, Tetramethylendiisocyanat-1,4 und vorzugsweise Hexamethylendiisocyanat-1,6, cycloaliphatische Diisocyanate, wie Cyclohexan-1,3- und -1,4-diisocyanat sowie beliebige Gemische dieser Isomeren, 1-lsocyanato-3,3,5-trimethyl-5-isocyanatomethylcyclohexan (IPDI), 2,4- und 2,6-Hexahydrotoluylendiisocyanat sowie die entsprechenden Isomerengemische, 4,4'-, 2,2'- und 2,4'-Dicyclohexylmethandiisocyanat sowie die entsprechenden Isomerengemische, und vorzugsweise aromatische Di- und Polyisocyanate, wie beispielsweise 2,4- und 2,6-Toluylendiisocyanat und die entsprechenden Isomerengemische, 4,4'-, 2,4'- und 2,2'-Diphenylmethandiisocyanat und die entsprechenden Isomerengemische, Mischungen aus 4,4'- und 2,2'-Diphenylmethandiisocyanaten, Polyphenylpolymethylenpolyisocyanate, Mischungen aus 4,4'-, 2,4'- und 2,2'-Diphenylmethandiisocyanaten und Polyphenylpolymethylenpolyisocyanaten (Roh-MDI) und Mischungen aus Roh-MDI und Toluylendiisocyanaten. Die organischen Di- und Polyisocyanate können einzeln oder in Form ihrer Mischungen eingesetzt werden.

Es ist auch möglich, Isocyanate einzusetzen, die durch den Einbau von Urethan-, Uretdion-, Isocyanurat-, Allophanat und anderen Gruppen modifiziert wurden, sogenannte modifizierte Isocyanate.

Besonders bewährt haben sich als organische Polyisocyanate und kommen daher vorzugsweise zur Anwendung:
Toluylendiisocyanat, Gemische aus Diphenylmethandiisocyanat-Isomeren, Gemische aus Diphenylmethandiisocyanat und Polyphenylpolymethylpolyisocyanat oder Toluylendiisocyanat mit Diphenylmethandiisocyanat und/oder Polyphenylpolymethylpolyisocyanat oder so genannte Präpolymere.

Es können sowohl TDI (2,4- und 2,6-Toluylendiisocyanat-Isomerengemisch) als auch MDI (4,4'-Diphenylmethandiisocyanat) verwendet werden. Das sogenannte "crude MDI" oder "polymere MDI" enthält neben dem 4,4'- auch die 2,4'- und 2,2'-Isomeren sowie höherkernige Produkte. Als "pure MDI" bezeichnet man zweikernige Produkte aus überwiegend 2,4'- und 4,4'-Isomerengemischen bzw. deren Präpolymere. Weitere geeignete Isocyanate sind in der Patentschrift EP 1095968 aufgeführt, auf die hier im vollen Umfang Bezug genommen wird.

Als Vernetzer werden niedermolekulare, gegenüber Isocyanaten reaktive mehrfunktionelle Verbindungen bezeichnet. Geeignet sind z.B. polyfunktionelle, insbesondere di- und trifunktionelle Verbindungen mit Molekulargewichten von 62 bis 1000 g/mol, vorzugsweise 62 bis 600 g/mol. Verwendung finden beispielsweise Di- und Trialkanolamine, wie Diethanolamin und Triethanolamin, aliphatische und aromatische Diamine, wie z. B. Ethylendiamin, Butylendiamin, Butylendiamin-1,4, Hexamethylendiamin-1,6, 4,4'-Diaminodiphenylmethan, 3,3'-dialkylsubstituierte 4,4'-Diaminodiphenylmethane, 2,4- und 2,6- Toluylendiamin und vorzugsweise aliphatische Diole und Triole mit 2 bis 6 Kohlenstoffatomen, wie Ethylenglykol, Propylenglykol, 1,4-Butylenglykol, 1,6-Hexamethylenglykol, 2-Methyl-1,3-propanediol Glycerin und Trimethylolpropan oder Ricinusöl oder Pentaerythrit, sowie höherwertige Alkohole, wie Zuckeralkohole, beispielsweise Saccharose, Glucose oder Sorbit und alkoxylierte Verbindungen aller zuvor genannten Beispiele.

Die Einsatzkonzentration liegt üblicherweise zwischen 0,1 und 5 Teilen, bezogen auf 100,0 Teile Polyol je nach Formulierung, kann aber auch davon abweichen. Bei Verwendung von MDI mit einer Funktionalität f > 2 bei der Formverschäumung übernimmt dies ebenfalls eine vernetzende Funktion. Der Gehalt an niedermolekularen Vernetzern kann daher bei steigender Menge an entsprechendem MDI reduziert werden.

Die erfindungsgemäßen Zusammensetzungen können in der Blockverschäumung eingesetzt werden. Es können alle dem Fachmann bekannten Verfahren zur Herstellung von frei gestiegenen Polyurethanweichschaumstoffen verwendet werden. So kann zum Beispiel der Schäumungsprozess sowohl in horizontaler als auch in vertikaler Richtung in diskontinuierlichen oder kontinuierlichen Anlagen erfolgen. Ebenso können die erfindungsgemäß einsetzbaren Additivzusammensetzungen für die CO₂-Technologie benutzt werden. Die Verwendung in Niederdruck- und Hochdruckmaschinen ist möglich, wobei die erfindungsgemäßen Formulierungen sowohl direkt in die Mischkammer dosiert werden können oder auch schon vor der Mischkammer einer der danach in die Mischkammer gelangenden Komponenten zugemischt werden. Die Zumischung kann auch im Rohstofftank erfolgen.

Als Polyolkomponente geeignete Polyole im Sinne der vorliegenden Erfindung sind alle organischen Substanzen mit mehreren gegenüber Isocyanaten reaktiven Gruppen, vorzugsweise OH-Gruppen, sowie deren Zubereitungen. Bevorzugte Polyole sind alle zur Herstellung von Polyurethansystemen, insbesondere Polyurethanschaumstoffen üblicherweise verwendeten Polyetherpolyole und Polyesterpolyole.

Es kann sich hierbei z. B. um Polyether- oder Polyesterpolyole handeln, die typischerweise 2 bis 8 OH-Gruppen pro Molekül tragen und neben Kohlenstoff, Wasserstoff und Sauerstoff auch Heteroatome wie Stickstoff, Phosphor oder Halogene enthalten können; vorzugsweise werden Polyetherpolyole eingesetzt. Solche Polyole können nach bekannten Verfahren hergestellt werden, beispielsweise durch anionische Polymerisation von Alkylenoxiden in Gegenwart von Alkalihydroxiden oder Alkalialkoholaten als Katalysatoren und unter Zusatz mindestens eines Startermoleküls, das bevorzugt 2 oder 3 reaktive Wasserstoffatome gebunden enthält oder durch kationische Polymerisation von Alkylenoxiden in Gegenwart von Lewis-Säuren wie beispielsweise Antimonpentachlorid oder Borfluorid-Etherat oder durch Doppelmetallcyanidkatalyse. Geeignete Alkylenoxide enthalten 2 bis 4 Kohlenstoffatome im Alkylenrest. Beispiele sind Tetrahydrofuran, 1,2-Propylenoxid, 1,2- bzw. 2,3-Butylenoxid; vorzugsweise werden Ethylenoxid und/oder 1,2-Propylenoxid eingesetzt. Die Alkylenoxide können einzeln, alternierend nacheinander oder als Mischungen verwendet werden. Als H-funktionelle Startsubstanzen kommen insbesondere mehrfunktionelle Alkohole und/oder Amine zum Einsatz. Bevorzugt eingesetzte Alkohole sind zweiwertige Alkohole, beispielsweise Ethylenglykol, Propylenglykol, oder Butandiole, dreiwertige Alkohole, beispielsweise Glycerin, Trimethylolpropan oder Ricinusöl oder Pentaerythrit, sowie höherwertige Alkohole, wie Zuckeralkohole, beispielsweise Saccharose, Glucose oder Sorbit. Bevorzugt eingesetzte Amine sind aliphatische Amine mit bis zu 10 Kohlenstoffatomen, beispielsweise Ethylendiamin, Diethylentriamin, Propylendiamin, aromatische Amine, beispielsweise Toluylendiamin oder Diaminodiphenylmethan, sowie Aminoalkohole, wie Ethanolamin oder Diethanolamin.

Polyesterpolyole können durch Polykondensationsreaktion oder durch ringöffnende Polymerisation dargestellt werden. Als Säurekomponenten werden z. B. Bernsteinsäure, Maleinsäure, Maleinsäureanhydrid, Adipinsäure, Phthalsäureanhydrid, Phthalsäure, Isophthalsäure, Terephthalsäure, Tetrahydrophtalsäure, Tetrahydrophthalsäureanhydrid, Hexahydrophthalsäureanhydrid oder Gemische aus den genannten Säuren und/oder Anhydriden eingesetzt. Als Alkoholkomponenten werden z. B. Ethandiol, 1,2-Propandiol, 1,3-Propandiol, 1,4-Butandiol. 1,5-Pentandiol, Neopentylglykol, 1,6-Hexandiol, 1,4-Bis(hydroxymethyl)cyclohexan, Diethylenglykol, Dipropylenglykol, Trimethylolpropan, Glycerin, Pentaerythrit oder Gemische aus den genannten Alkoholen verwendet. Werden als Alkoholkomponente zweiwertige oder mehrwertige Polyetherpolyole eingesetzt, so erhält man Polyesteretherpolyole die ebenfalls als Startersubstanzen zur Herstellung der Polyetherpolycarbonatpolyole dienen können. Bevorzugt werden Polyetherpolyole mit Mn = 150 bis 2000 g/mol zur Herstellung der Polyesteretherpolyole eingesetzt.

Die Polyetherpolyole, vorzugsweise Polyoxypropylenpolyoxyethylenpolyole besitzen typischerweise eine Funktionalität von 2 bis 8 und zahlengemittelte Molekulargewichte vorzugsweise im Bereich von 500 bis 8000, vorzugsweise 800 bis 4500. Weitere Polyole sind dem Fachmann bekannt und können zum Beispiel der EP-A-0380993 oder US-A-3346557 entnommen werden, auf die im vollen Umfang Bezug genommen wird.

Bevorzugt werden zur Herstellung von hochelastischen Polyurethan-Weichschaumstoffen (Kaltschaum) zwei- und/oder dreifunktionelle Polyetheralkohole eingesetzt, die bevorzugt über 50 mol-% primäre Hydroxylgruppen bezogen auf die Summe der Hydroxylgruppen aufweisen, insbesondere solche mit einem Ethylenoxidblock am Kettenende oder solche, die nur auf Ethylenoxid basieren.

Bevorzugt werden zur Herstellung von Blockweichschaumstoffen zwei- und/oder dreifunktionelle Polyetheralkohole eingesetzt, die sekundäre Hydroxylgruppen, bevorzugt über 80 mol-%, aufweisen, insbesondere solche mit einem Propylenoxidblock oder statistischen Propylen- und Ethylenoxidblock am Kettenende oder solche, die nur auf Propylenoxidblöcken basieren.

Eine weitere Klasse von Polyolen sind solche, die als Präpolymere durch Umsetzung von Polyol mit Isocyanat in einem Molverhältnis von 100 zu 1 bis 5 zu 1, bevorzugt 50 zu 1 bis 10 zu 1 erhalten werden. Solche Präpolymere werden vorzugsweise gelöst in Polyol eingesetzt, wobei das Polyol bevorzugt dem zur Herstellung der Präpolymeren eingesetzten Polyol entspricht.

Noch eine weitere Klasse von Polyolen stellen die sogenannten Füllkörperpolyole (Polymerpolyole) dar. Diese zeichnen sich dadurch aus, dass sie feste organische Füllstoffe bis zu einem Feststoffgehalt von 40 Gew.-% oder mehr in disperser Verteilung enthalten. Man verwendet unter anderem:
SAN-Polyole: Dies sind hochreaktive Polyole, welche ein Copolymer auf der Basis Styrol/Acrylnitril (SAN) dispergiert enthalten.
PHD-Polyole: Dies sind hochreaktive Polyole, welche Polyharnstoff ebenfalls in dispergierter Form enthalten.
PIPA-Polyole: Dies sind hochreaktive Polyole, welche ein Polyurethan, beispielsweise durch in situ-Reaktion eines Isocyanats mit einem Alkanolamin in einem konventionellen Polyol gebildet, in dispergierter Form enthalten.

Der Festkörperanteil, der je nach Anwendung bevorzugt zwischen 5 und 40 Gew.-%, bezogen auf das Polyol liegt, ist für eine verbesserte Zellöffnung verantwortlich, so dass das Polyol insbesondere mit TDI kontrolliert verschäumbar wird und kein Schrumpfen der Schäume auftritt. Der Festkörper wirkt damit als wesentliche Prozesshilfe. Eine weitere Funktion besteht darin, über den Feststoffanteil die Härte zu kontrollieren, denn höhere Festkörperanteile bewirken eine höhere Härte des Schaums.

Die Formulierungen mit feststoffhaltigen Polyolen sind deutlich weniger eigenstabil und bedürfen daher neben der chemischen Stabilisierung durch die Vernetzungsreaktion eher auch zusätzlich einer physikalischen Stabilisierung.

Je nach Feststoffgehalt der Polyole werden diese alleine oder in Abmischung mit den oben genannten ungefüllten Polyolen eingesetzt.

Eine weitere Klasse von einsetzbaren Polyolen stellen die sogenannten autokatalytischen Polyole, insbesondere autokatalytische Polyetherpolyole, dar. Solche Polyole basieren zum Beispiel auf Polyether-Blöcken, vorzugsweise auf Ethylenoxid- und/oder Propylenoxid-Blöcken, und beinhalten zudem katalytisch aktive funktionelle Gruppen, wie zum Beispiel stickstoffhaltige funktionelle Gruppen, insbesondere Amino-Gruppen, vorzugsweise tertiäre Amin-Funktionen, HarnstoffGruppen und/oder Heterocyclen enthaltend Stickstoff-Atome. Durch die Verwendung solcher autokatalytsichen Polyole bei der Herstellung von Polyurethansystemen, insbesondere von Polyurethanschaumstoffen, bevorzugt von Polyurethanweichschaumstoffen, kann die benötigte Menge an ggf. verwendeten Katalysatoren je nach Anwendung gegebenenfalls reduziert und/oder auf spezielle gewünschte Schaumeigenschaften angepasst werden. Geeignete Polyole sind zum Beispiel in WO0158976 (A1), WO2005063841 (A1), WO0222702 (A1), WO2006055396 (A1), WO03029320 (A1), WO0158976 (A1), US6924321 (B2), US6762274 (B2), EP2104696 (B1), WO2004060956 (A1) oder WO2013102053 (A1) beschrieben und können zum Beispiel unter den Handelsnamen Voractiv™ und/oder SpecFlex™ Activ bei der Firma Dow bezogen werden.

Als Treibmittel können die bekannten Treibmittel eingesetzt werden. Vorzugsweise werden bei der Herstellung des Polyurethanschaums Wasser, Methylenchlorid, Pentan, Alkane, halogenierte Alkane, Aceton und/oder Kohlendioxid als Treibmittel eingesetzt.

Das Wasser kann der Mischung direkt zugegeben werden oder aber als Nebenkomponente eines der Edukte, wie z. B. der Polyolkomponente, mit dieser der Mischung zugegeben werden.

Neben physikalischen Treibmitteln und ggf. Wasser können auch andere chemische Treibmittel eingesetzt werden, die mit Isocyanaten unter Gasentwicklung reagieren, wie beispielsweise Ameisensäure.

Als Katalysatoren können im Rahmen dieser Erfindung zum Beispiel jegliche Katalysatoren für die Reaktionen Isocyanat-Polyol (Urethan-Bildung) und/oder Isocyanat-Wasser (Amin- und Kohlenstoffdioxid-Bildung) und/oder die Isocyanat-Dimerisierung (Uretdion-Bildung) Isocyanat-Trimerisierung (Isocyanurat-Bildung), Isocyanat-Isocyanat mit CO₂-Abspaltung (Carbodiimid-Bildung) und/oder Isocyanat-Amin (Harnstoff-Bildung) und/oder "sekundäre" Vernetzungsreaktionen wie Isocyanat-Urethan (Allophanat-Bildung) und/oder Isocyanat-Harnstoff (Biuret-Bildung) und/oder Isocyanat-Carbodiimid (Uretonimin-Bildung) eingesetzt werden.

Geeignete Katalysatoren im Sinne der vorliegenden Erfindung sind beispielsweise Substanzen, die eine der vorgenannten Umsetzungen, insbesondere die Gelreaktion (Isocyanat-Polyol), die Treibreaktion (Isocyanat-Wasser) und/oder die Di- bzw. Trimerisierung des Isocyanats katalysieren. Solche Katalysatoren sind vorzugsweise stickstoffhaltige Verbindungen, insbesondere Amine und Ammonium-Salze, und/oder metallhaltige Verbindungen.

Geeignete stickstoffhaltige Verbindungen als Katalysatoren, im Folgenden auch als stickstoffhaltige Katalysatoren bezeichnet, im Sinne der vorliegenden Erfindung sind alle stickstoffhaltigen Verbindungen nach dem Stand der Technik, die eine der oben genannten Isocyanat-Reaktionen katalysieren und/oder zur Herstellung von Polyurethanen, insbesondere von Polyurethanschaumstoffen eingesetzt werden können.

Beispiele für geeignete stickstoffhaltige Verbindungen als Katalysatoren im Sinne der vorliegenden Erfindung sind bevorzugt Amine, insbesondere tertiäre Amine oder Verbindungen enthaltend eine oder mehrere tertiäre Amin-Gruppen, wie unter anderem die Amine Triethylamin, N,N-Dimethylcyclohexylamin, N,N-Dicyclohexylmethylamin, N,N-Dimethylaminoethylamin, N,N,N',N'-Tetramethylethylen-1,2-diamin, N,N,N',N'-Tetramethylpropylen-1,3-diamin, N,N,N',N'-Tetramethyl-1,4-butandiamin, N,N,N',N'-Tetramethyl-1,6-hexandiamin, N,N,N',N",N"-Pentamethyldiethylentriamin, N,N,N'-Trimethylaminoethylethanolamin, N,N-Dimethylaminopropylamin, N,N-Diethylaminopropylamin, N,N-Dimethylaminopropyl-N,N-dipropan-2-olamin, 2-[[3-(dimethylamino)propyl]-methylamino]ethanol, 3-(2-Dimethylamino)ethoxy)propylamin, N,N-Bis[3-(dimethylamino)propyl]-amin, N,N,N',N",N"-Pentamethyldipropylentriamin, 1-[Bis[3-(dimethylamino)propyl]amino]-2-propanol, N,N-Bis[3-(dimethylamino)propyl]-N',N'-dimethylpropan-1,3-diamin, Triethylendiamin, 1,4-Diazabicyclo[2.2.2]octane-2-methanol, N,N'-Dimethylpiperazin, 1,2-Dimethylimidazol, N-(2-Hydroxypropyl)imidazol, 1-Isobutyl-2-methylimidazol, N-(3-Aminopropyl)imidazol, N-Methylimidazol, N-Ethylmorpholin, N-Methylmorpholin, 2,2,4-Trimethyl-2-silamorpholin, N-Ethyl-2,2-dimethyl-2-silamorpholin, N-(2-Aminoethyl)morpholin, N-(2-Hydroxyethyl)morpholin, 2,2'-Dimorpholinodiethylether, N,N'-Dimethylpiperazin, N-(2-Hydroxyethyl)piperazin, N-(2-Aminoethyl)-piperazin, N,N-Dimethylbenzylamin, N,N-Dimethylaminoethanol, N,N-Diethylaminoethanol, 3-Dimethylamino-1-propanol, N,N-Dimethylaminoethoxyethanol, N,N-Diethylaminoethoxyethanol, Bis(2-Dimethylaminoethylether), N,N,N'-Trimethyl-N'-(2-hydroxyethyl)bis(2-aminoethyl)ether, N,N,N'-Trimethyl-N-3'-aminopropyl(bisaminoethyl)ether, Tris(dimethylaminopropyl)hexahydro-1,3,5-triazin, 1,8-Diazabicyclo[5.4.0]undec-7-en, 1,5-Diazabicyclo[4.3.0]non-5-en, 1,5,7-triazabicyclo[4.4.0]dec-5-en, N-Methyl-1,5,7-triazabicyclo[4.4.0]dec-5-en, 1,4,6-triazabicyclo[3.3.0]oct-4-en, 1,1,3,3-Tetramethylguanidin, tert-Butyl-1,1,3,3-Tetramethylguanidin, Guanidin, 3-Dimethylaminopropylharnstoff, 1,3-Bis[3-(dimethylamino)propyl]harnstoff, Bis-N,N-(dimethylaminoethoxyethyl)isophorondicarbamat, 3-Dimethylamino-N,N-dimethylpropionamid und 2,4,6-Tris(dimethylaminomethyl)phenol. Geeignete stickstoffhaltige Katalysatoren, nach dem Stand der Technik, können zum Beispiel von der Firma Evonik unter dem Handelsnamen TEGOAMIN® bezogen werden.

Je nach Anwendung kann es bevorzugt sein, dass bei der erfindungsgemäßen Herstellung von Polyurethanschaumstoffen, quaternisierte und/oder protonierte stickstoffhaltige Katalysatoren, insbesondere quaternisierte und/oder protonierte tertiäre Amine, eingesetzt werden.

Zur möglichen Quaternisierung stickstoffhaltiger Katalysatoren können alle als Quaternisierungsreagenz bekannten Reagenzien eingesetzt werden. Vorzugsweise werden als Quaternisierungsmittel Alkylierungsmittel, wie z. B. Dimethylsulfat, Methylchlorid oder Benzylchlorid, bevorzugt Methylierungsmittel wie insbesondere Dimethylsulfat verwendet. Ebenso kann mit Alkylenoxiden wie zum Beispiel Ethylenoxid, Propylenoxid oder Butylenoxid, vorzugsweise mit anschließender Neutralisation mit anorganischen oder organischen Säuren, quaternisiert werden.

Stickstoffhaltige Katalysatoren können, sofern quaternisiert, einfach oder mehrfach quaternisiert sein. Vorzugsweise sind die stickstoffhaltigen Katalysatoren nur einfach quaternisiert. Bei einfacher Quaternisierung sind die stickstoffhaltigen Katalysatoren vorzugsweise an einem tertiären Stickstoffatom quaternisiert.

Stickstoffhaltige Katalysatoren können durch Umsetzung mit organischen oder anorganischen Säuren in die entsprechenden protonierten Verbindungen überführt werden. Diese protonierten Verbindungen können z.B. bevorzugt sein, wenn z.B. eine verlangsamte Polyurethan-Reaktion erzielt werden soll oder wenn das Reaktionsgemisch bei der Anwendung ein verbessertes Fließverhalten haben soll.

Als organische Säuren können zum Beispiel alle nachfolgend genannten organischen Säuren, beispielsweise Carbonsäuren mit 1 bis 36 Kohlenstoffatomen (aromatisch oder aliphatisch, linear oder verzweigt), beispielsweise Ameisensäure, Milchsäure, 2-Ethylhexansäure, Salicylsäure und Neodecansäure, oder auch polymere Säuren wie z.B. Polyacryl- oder Polymethacrylsäuren eingesetzt werden. Als anorganische Säuren können z.B. Phosphor-basierende Säuren, Schwefelbasierende Säuren oder Bor-basierende Säuren eingesetzt werden.

Der Einsatz stickstoffhaltigen Katalysatoren, die nicht quaternisiert oder protoniert sind, ist im Sinne dieser Erfindung allerdings besonders bevorzugt.

Geeignete metallhaltige Verbindungen als Katalysatoren, im Folgenden auch als metallhaltige Katalysatoren bezeichnet, im Sinne der vorliegenden Erfindung sind alle metallhaltigen Verbindungen nach dem Stand der Technik, die eine der oben genannten Isocyanat-Reaktionen katalysieren und/oder zur Herstellung von Polyurethanen, insbesondere von Polyurethanschaumstoffen eingesetzt werden können. Sie können zum Beispiel ausgewählt werden aus der Gruppe der metallorganischen oder organometallischen Verbindungen, metallorganischen oder organometallischen Salze, organischen Metallsalze, anorganischen Metallsalze sowie aus der Gruppe der geladenen oder ungeladenen metallhaltigen Koordinationsverbindungen, insbesondere der Metall-Chelat-Komplexe.

Der Ausdruck "metallorganische oder organometallische Verbindungen" umfasst im Sinne dieser Erfindung insbesondere den Einsatz metallhaltiger Verbindungen, die über eine direkte Kohlenstoff-Metall-Bindung verfügen, hier auch als Metallorganyle (z.B. Zinnorganyle) oder organometallische bzw. Organometall-Verbindungen (z.B. Organozinn-Verbindungen) bezeichnet. Der Ausdruck "organometallische oder metallorganische Salze" umfasst im Sinne dieser Erfindung insbesondere den Einsatz von metallorganischen oder organometallischen Verbindungen mit Salzcharakter, das heißt Ionenverbindungen, bei denen entweder das Anion oder Kation von metallorganischer Natur ist (z.B. Organozinn-Oxide, Organozinn-Chloride oder Organozinn-Carboxylate). Der Ausdruck "organische Metallsalze" umfasst im Sinne dieser Erfindung insbesondere den Einsatz von metallhaltigen Verbindungen, die über keine direkte Kohlenstoff-Metall-Bindung verfügen und gleichzeitig Metallsalze sind, bei denen entweder das Anion oder das Kation eine organische Verbindung ist (z.B. Zinn(II)-Carboxylate). Der Ausdruck "anorganische Metallsalze" umfasst im Sinne dieser Erfindung insbesondere den Einsatz von metallhaltigen Verbindungen oder von Metallsalzen, bei denen weder Anion noch Kation eine organische Verbindung ist, z.B. Metall-Chloride (z.B. Zinn(II)-Chlorid), reine oder gemischte, also mehrere Metalle enthaltende, Metall-Oxide (z.B. Zinn-Oxide) und/oder Metall-Silicate oder -Alumosilicate. Der Ausdruck "Koordinationsverbindung" umfasst im Sinne dieser Erfindung insbesondere den Einsatz von metallhaltigen Verbindungen, die aus einem oder mehreren Zentralteilchen und einem oder mehreren Liganden aufgebaut sind, wobei die Zentralteilchen geladene oder ungeladene Metalle sind (z.B. Metall- bzw. Zinn-Amin-Komplexe). Der Ausdruck "Metall-Chelat-Komplexe" umfasst im Sinne dieser Erfindung insbesondere den Einsatz von metallhaltigen Koordinationsverbindungen, die Liganden mit mindestens zwei Koordinations- oder Bindungsstellen zum Metallzentrum aufweisen (z.B. Metall- bzw. Zinn-Polyamin- oder Metall- bzw. Zinn-Polyether-Komplexe).

Geeignete metallhaltige Verbindungen, insbesondere wie oben definiert, als Katalysatoren im Sinne der vorliegenden Erfindung können zum Beispiel ausgewählt werden aus allen metallhaltigen Verbindungen enthaltend Lithium, Natrium, Kalium, Magnesium, Calcium, Scandium, Yttrium, Titan, Zirconium, Vanadium, Niob, Chrom, Molybdän, Wolfram, Mangan, Cobalt, Nickel, Kupfer, Zink, Quecksilber, Aluminium, Gallium, Indium, Germanium, Zinn, Blei, und/oder Bismuth, insbesondere Natrium, Kalium, Magnesium, Calcium, Titan, Zirconium, Molybdän, Wolfram, Zink, Aluminium, Zinn und/oder Bismuth, besonders bevorzugt Zinn, Bismuth, Zink und/oder Kalium.

Geeignete metallorganische Salze und organische Metallsalze, wie oben definiert, als Katalysatoren im Sinne der vorliegenden Erfindung sind zum Beispiel Organozinn-, Zinn-, Zink-, Bismuth und Kalium-Salze, insbesondere entsprechende Metall-Carboxylate, -Alkoholate, -Thiolate und -Mercaptoacetate, wie zum Beispiel Dibutylzinndiacetat, Dimethylzinndilaurat, Dibutylzinndilaurat (DBTDL), Dioctylzinndilaurat (DOTDL), Dimethylzinndineodecanoat, Dibutylzinndineodecanoat, Dioctylzinndineodecanoat, Dibutylzinndioleat, Dibutylzinn-bis-n-laurylmercaptid, Dimethylzinn-bis-n-laurylmercaptid, Monomethylzinn-tris-2-ethylhexylmercaptoacetat, Dimethylzinn-bis-2-ethylhexylmercaptoacetat, Dibutylzinn-bis-2-ethylhexylmercaptoacetat, Dioctylzinn-bis-isooctylmercaptoacetat, Zinn(II)-acetat, Zinn(II)-2-ethylhexanoat (Zinn(II)-octoat), Zinn(II)-isononanoat (Zinn(II)-3,5,5-trimethylhexanoat), Zinn(II)-neodecanoat, Zinn(II)-ricinoleat, Zinn(II)-oleat, Zink(II)-acetat, Zink(II)-2-ethylhexanoat (Zink(II)-octoat), Zink(II)-isononanoat (Zink(II)-3,5,5-trimethylhexanoat), Zink(II)-neodecanoat, Zink(II)-ricinoleat, Bismuthacetat, Bismuth-2-ethylhexanoat, Bismuthoctoat, Bismuthisononanoat, Bismuthneodecanoat, Kaliumformiat, Kaliumacetat, Kalium-2-ethylhexanoat (Kaliumoctoat), Kalium-isononanoat, Kalium-neodecanoat und/oder Kaliumricinoleat.

Bei der erfindungsgemäßen Herstellung von Polyurethanschaumstoffen, kann es bevorzugt sein, die Verwendung von metallorganischen Salzen wie zum Beispiel von Dibutylzinndilaurat auszuschließen.

Geeignete metallhaltige Katalysatoren werden in der Regel vorzugsweise so ausgewählt, dass sie keinen störenden Eigengeruch aufweisen, toxikologisch im Wesentlichen unbedenklich sind und dass die resultierenden Polyurethansysteme, insbesondere Polyurethanschäume möglichst geringe Katalysator-bedingte Emissionen aufweisen.

Bei der erfindungsgemäßen Herstellung von Polyurethanschaumstoffen, kann es je nach Art der Anwendung, bevorzugt sein, einbaubare/reaktive oder hochmolekulare Katalysatoren zu verwenden. Solche Katalysatoren können zum Beispiel ausgewählt werden aus der Gruppe der metallhaltigen Verbindungen, vorzugsweise aus der Gruppe der Zinn-, Zink-, Bismuth- und/oder Kalium-haltigen Verbindungen, insbesondere aus der Gruppe der Metall-Carboxylate der vorgenannten Metalle wie zum Beispiel die Zinn-, Zink-, Bismuth- und/oder Kaliumsalze der Isononansäure, Neodecansäure, Ricinolsäure und/oder Ölsäure, und/oder aus der Gruppe der stickstoffhaltigen Verbindungen, insbesondere aus der Gruppe der emissionsarmen Amine und/oder der emissionsarmen Verbindungen enthaltend eine oder mehrere tertiäre Amine-Gruppen, beispielsweise beschrieben durch die Amine Dimethylaminoethanol, N,N-Dimethyl-N',N'-di(2-hydroxypropyl)-1,3-diaminopropan, N,N-Dimethylaminopropylamin, N,N,N'-Trimethyl-N'-hydroxyethylbis(aminoethyl)ether, 6-Dimethylaminoethyl-1-hexanol, N-(2-Hydroxypropyl)imidazol, N-(3-Amino-propyl)imidazol, Aminopropyl-2-methylimidazol, N,N,N'-Trimethylaminoethanolamin, 2-(2-(N,N-Dimethylaminoethoxy)ethanol, N-(Dimethyl-3-aminopropyl)harnstoff-derivate und Alkylaminooxamide, wie Bis-(N-(N',N'-dimethylaminopropyl))oxamid, Bis-(N-(N',N'-dimethylaminoethyl))-oxamid, Bis-(N-(N',N'-Imidazolidinylpropyl)oxamid, Bis-(N-(N',N'-diethylaminoethyl))-oxamid, Bis-(N-(N',N'-diethylaminopropyl)oxamid, Bis-(N-(N',N'-diethylaminoethyl)oxamid, Bis-(N-(N',N'-diethylimino-1-methylpropyl)oxamid, Bis-(N-(3-Morpholinopropylyl)-oxamid und deren Umsetzungsprodukte mit Alkylenoxiden, vorzugsweise mit einem Molgewicht im Bereich zwischen 160 und 500 g/mol, sowie Verbindungen der allgemeine Formel: 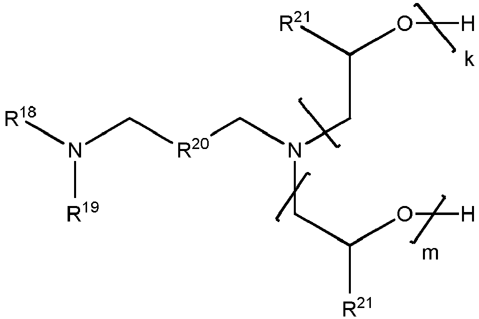 mit
R18, R19 = -CₐH₂ₐ₊ᵢ mit a = 1 - 4 für acylische Gruppen
R18, R19 = -C_{b}H_{c}N_{d}- mit b = 3 - 7, c = 6 - 14, d = 0 - 2 für cyclische Gruppen
R20 = CₑH_{f}O₉ mit e = 0 - 4, f= 0 - 8, g = 0 - 2
R21 = -H, -CH₃, -C₂H₅
k, m = gleich oder verschieden 1 - 5.

Derartige Katalysatoren und/oder Mischungen werden beispielsweise unter dem Namen Jeffcat® ZF-10, Lupragen® DMEA, Lupragen® API, Toyocat® RX 20 und Toyocat® RX 21 , DABCO® RP 202, DABCO® RP 204, DABCO® NE 300, DABCO® NE 310, DABCO® NE 400, DABCO® NE 500, DABCO® NE 600, DABCO® NE 1060 und DABCO® NE 2039, Niax® EF 860, Niax® EF 890, Niax® EF 700, Niax® EF 705, Niax® EF 708, Niax® EF 600, Niax® EF 602, Kosmos® 54, Kosmos® EF, und Tegoamin® ZE 1 im Handel angeboten.

Geeignete Einsatzmengen an Katalysatoren richten sich nach dem Typ des Katalysators und liegen vorzugsweise im Bereich von 0,005 bis 10,0 pphp, besonders bevorzugt im Bereich von 0,01 bis 5,00 pphp (= Gewichtsteile bezogen auf 100 Gewichtsteile Polyol) bzw. 0,10 bis 10,0 pphp für Kaliumsalze.

Je nach Anwendung kann es bevorzugt sein, dass bei der erfindungsgemäßen Herstellung von Polyurethanschaumstoffen, ein oder mehrere stickstoffhaltige und/oder metallhaltige Katalysatoren eingesetzt werden. Wenn mehr als ein Katalysator eingesetzt wird, können die Katalysatoren in beliebigen Gemischen untereinander eingesetzt werden. Hierbei können die Katalysatoren einzeln während der Verschäumung, zum Beispiel im Sinne einer Vordosierung im Mischkopf, und/oder als vorgemischte Katalysatorkombination eingesetzt werden.

Der Ausdruck "vorgemischte Katalysatorkombination", im Folgenden auch als Katalysatorkombination bezeichnet, umfasst im Sinne dieser Erfindung insbesondere fertige Mischungen von metallhaltigen Katalysatoren und/oder stickstoffhaltigen Katalysatoren und/oder entsprechenden protonierten und/oder quaternisierten stickstoffhaltigen Katalysatoren sowie optional noch weiterer Inhalts- oder Zusatzstoffen wie zum Beispiel Wasser, organischen Lösungsmittelen, Säuren zur Blockierung der Amine, Emulgatoren, Tenside, Treibmitteln, Antioxidantien, Flammschutzmittel, Schaumstabilisatoren und/oder Siloxanen, vorzugsweise Polyethersiloxanen, die bereits vor der Verschäumung als solche vorliegen und während der Verschäumungsvorgangs nicht als Einzelkomponenten zugegeben werden müssen.

Je nach Anwendung kann es bevorzugt sein, wenn aus der Summe aller eingesetzten stickstoffhaltigen Katalysatoren, gegenüber der Summe der metallhaltigen Katalysatoren, insbesondere Kalium-, Zink- und/oder Zinn-Katalysatoren, ein molares Mengenverhältnis von 1:0,05 bis 0,05:1, vorzugsweise 1:0,07 bis 0,07:1 und besonders bevorzugt 1:0,1 bis 0,1:1 resultiert.

Um eine Reaktion der Komponenten untereinander zu vermeiden, insbesondere Reaktion von stickstoffhaltigen Katalysatoren mit metallhaltigen Katalysatoren, insbesondere Kalium-, Zink- und/oder Zinn-Katalysatoren, kann es bevorzugt sein, diese Komponenten getrennt voneinander zu lagern und dann der Isocyanat- und Polyol-Reaktionsmischung gleichzeitig oder nacheinander zuzuführen.

Mittels des erfindungsgemäßen Verfahrens ist ein Polyurethansystem, vorzugsweise Polyurethanschaum, insbesondere ein Polyurethanweichschaum erhältlich. Dieses Polyurethansystem entspricht einem weiteren Gegenstand der Erfindung. Der betreffende Polyurethanschaum zeichnet sich insbesondere dadurch aus, dass er aufgrund der Verwendung des erfindungsgemäßen Additivs als Antioxidans besonders emissionsarm ist.

Es entspricht einer bevorzugten Ausführungsform der Erfindung, wenn das Polyurethansystem 0,0001 bis 10 Gew.-%, vorzugsweise 0, 001 bis 5 Gew.-%, insbesondere 0,01 bis 3 Gew.-%, bezogen auf das Gesamtgewicht des Polyurethansystems, einer oder mehrerer Verbindungen der Formel (I) oder einer Antioxidationsmittelmischung, wie zuvor beschrieben, enthält.

Mit dem erfindungsgemäßen Polyurethansystem, insbesondere Polyurethanschaum, sind Artikel zugänglich die dieses Polyurethansystem, insbesondere Polyurethanschaum, enthalten oder aus ihm bestehen. Diese Artikel stellen einen weiteren Gegenstand dieser Erfindung dar. Solche Artikel können z. B. Möbelpolster oder Matratzen sein.

Im Rahmen dieser Erfindung wird außerdem ein Polyurethansystem offenbart, enthaltend die Umsetzungsprodukte einer oder mehrerer Polyolkomponenten, mit einer oder mehrerer Isocyanatkomponenten, wobei ein Hydroxyphenylcarbonsäureester der Formel (I) 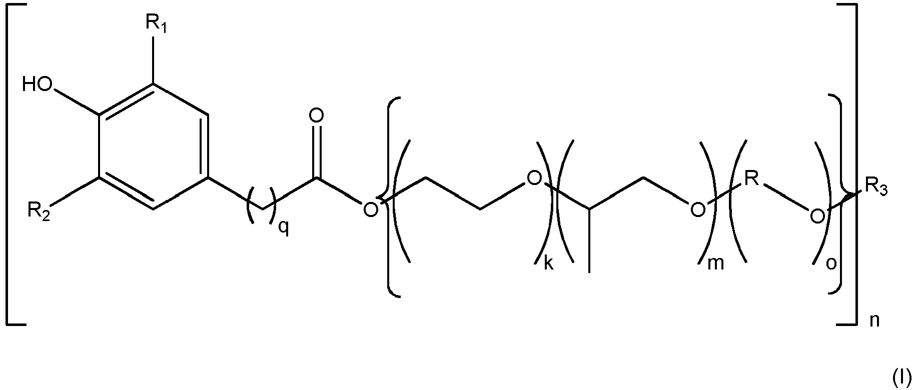 worin
- R: CH₂-CH(R^{I}), CH(R^{II})-CH(R^{II}), CH₂-C(R^{II})₂, C(R^{II})₂-C(R^{II})₂, 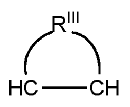 CH₂-CH-CH₂-R^{IV}, C₆H₆-CH-CH₂, oder C₆H₆-C(CH₃)-CH₂, mit
- R^{I}: C₂ bis C₂₄-Alkylrest oder Alkenrest, der linear oder verzweigt sein kann
- R^{II}: C₂ bis C₂₄-Alkylrest, Alkenrest, der linear oder verzweigt sein kann
- R^{III}: C₃ bis C₆ Alkylrest, der linear angeordnet ist, und
- R^{IV}: OH, Cl, OCH₃, OCH₂-CH₃, O-CH₂-CH=CH₂, O-CH=CH₂, Molekülrest von ein- oder mehrfach epoxidierten Fetten oder Ölen als Mono-, Di-, und Triglyceride oder Molekülrest von ein- oder mehrfach epoxidierten Fettsäuren oder deren C₁-C₂₄-Alkylestern,
- R₁ und R₂: unabhängig voneinander geradkettig oder verzweigt C₁-C₈-Alkyl, Cyclopentyl oder Cyclohexyl, insbesondere tert.-Butyl,
- q: 1, 2 oder 3, vorzugsweise 2 oder 3, insbesondere 2,
- n: eine ganze Zahl von 1 bis 30, vorzugsweise eine ganze Zahl von 1 bis 10, vorteilhafterweise 1,2,3,4,5 oder 6, z.B. 1,2,3 oder 4, insbesondere 1,
- R₃: einen n-wertigen Rest von linearem oder verzweigtem C₁-C₃₀-Alkyl, C₂-C₃₀-Alkylen, welche jeweils gegebenenfalls durch ein oder mehrere Sauerstoffatome unterbrochen sind, oder (für n = 1-12) einen n-wertigen Rest von C₅-C₁₂-Cycloalkyl, oder einen Rest R₄-[NR₅-C_{q}H_{2q}-]ₚ,
- R₄: Wasserstoff, einen n-wertigen Rest von linearem oder verzweigtem C₁-C₃₀-Alkyl, welches gegebenenfalls durch eine oder mehrere Gruppen -NR₅- unterbrochen ist, oder (für n = 1-12) einen n-wertigen Rest von C₅-C₁₂-Cycloalkyl,
- R₅: unabhängig voneinander Wasserstoff oder Methyl oder -C_{q}H_{2q}-, vorzugsweise Wasserstoff, und
- p: derjenigen Anzahl -[NR₅-C_{q}H_{2q}-]-Gruppen entspricht, welche n Reste -C_{q}H_{2q}- pro Molekül ergibt,
- k: eine ganze Zahl zwischen 0 und 50, vorzugsweise zwischen 10 und 30,
- m: eine ganze Zahl zwischen 0 und 50, z.B. 1-40, und
- o: eine ganze Zahl zwischen 0 und 50, vorzugsweise zwischen 0 und 30, insbesondere 0 darstellt,
- mit: (k + m + o) > 10
als ein Alterungsschutzmittel für synthetische Polymere, die gegen oxidativen, thermischen oder durch Licht induzierten Abbau empfindlich sind, zum Einsatz kommt.

Gilt in der Formel (I) k, m, o > 0 oder k, m > 0 und gleichzeitig o = 0, so ist die Reihenfolge der Monomerbausteine Ethylenoxid, Propylenoxid und (R-Oxid) in den einzelnen Polymerketten 1 bis n beliebig und k, m und o stellen Mittelwerte dar. Außerdem können die einzelnen Bausteine (EO), (PO) und (RO) entweder in Form von Blöcken, streng alternierend oder in Form von Gradienten aneinander gebunden sein.

Eine bevorzugte erfindungsgemäße Zusammensetzung zur Herstellung eines Polyurethansystems, insbesondere von Polyurethanschaum, kann Polyol in Mengen von 25 bis 80 Gew.-%, Wasser in Mengen von 1 bis 5 Gew.-%, Katalysator in Mengen von 0,05 bis 1 Gew.-%, physikalisches Treibmittel in Mengen von 0 bis 25 Gew.-% (z.B. 0,1 bis 25 Gew.-%), Schaumstabilisatoren (wie z. B. Si-haltige und nicht Si-haltige, insbesondere Si-haltige und nicht Si-haltige organische Stabilisatoren und Tenside) in Mengen von 0,1 bis 5 Gew.-%, Isocyanat in Mengen von 20 bis 50 Gew.% und das erfindungsgemäß einzusetzende Additiv, also Verbindungen der Formel (I), oder eine erfindungsgemäße Antioxidationsmittelmischung, in Mengen von 0,0001 bis 10 Gew.-%, vorzugsweise 0,001 bis 5 Gew-%, insbesondere 0,01 bis 3 Gew.-%, bezogen auf das Gesamtgewicht des Polyurethansystems, enthalten. Die Verbindung der Formel (I) gelangt insbesondere als erfindungsgemäße Antioxidationsmittelmischung zum Einsatz, umfassend Verbindungen der Formel (I) sowie vorzugsweise Verbindungen der Formel (II), insbesondere umfassend Verbindungen der Formel (I) sowie Verbindungen der Formel (II) und (III).

Bezüglich bevorzugter Ausführungsformen dieser vorgenannten Zusammensetzungen wird ausdrücklich auf die vorangegangene Beschreibung verwiesen.

Ein weiterer Gegenstand der Erfindung ist die Verwendung der erfindungsgemäß erhältlichen Polyurethansysteme als Kühlschrankisolierung, Dämmplatte, Sandwichelement, Rohrisolation, Sprühschaum, 1- & 1,5-Komponenten-Dosenschaum, Holzimitat, Modellschaum, Verpackungsschaum, Matratze, Möbelpolster, Werkstoff in Kraftfahrzeuginnenräumen, Automobil-Sitzpolster, Kopfstütze, Instrumententafel, Automobil-Innenverkleidung, Automobil-Dachhimmel, Schallabsorptionsmaterial, Lenkrad, Schuhsole, Teppichrückseitenschaum, Filterschaum, Dichtschaum, Dichtmittel und Kleber oder zur Herstellung entsprechender Produkte, insbesondere als Werkstoff in Kraftfahrzeuginnenräumen.

Ein weiterer Gegenstand der Erfindung ist die Verwendung von Verbindungen der Formel (I) oder Antioxidationsmittelmischungen, wie zuvor beschrieben, zur Herstellung von emissionsarmen Polyurethansystemen, insbesondere PUR-Schaum, mit einem verringerten Wert für VOC und das Fogging. Die Verbindung der Formel (I) gelangt dabei als erfindungsgemäße Antioxidationsmittelmischung zum Einsatz, umfassend Verbindungen der Formel (I) sowie Verbindungen der Formel (II), insbesondere umfassend Verbindungen der Formel (I) sowie Verbindungen der Formel (II) und (III),

Ein weiterer Gegenstand der Erfindung ist die Verwendung von Verbindungen der Formel (I) oder Antioxidationsmittelmischungen, wie zuvor beschrieben, zur Herstellung von geruchsarmen Polyurethansystemen, insbesondere PUR-Schaum. Die Verbindung der Formel (I) gelangt als erfindungsgemäße Antioxidationsmittelmischung zum Einsatz, umfassend Verbindungen der Formel (I) sowie Verbindungen der Formel (II), insbesondere umfassend Verbindungen der Formel (I) sowie Verbindungen der Formel (II) und (III),

Auch wird ein Verfahren zur Erniedrigung der Gesamtemission organischer Verbindungen aus Polyurethansystemen, insbesondere Polyurethanschaumstoffen, offenbart, durch Zugabe von Verbindungen gemäß Formel (I) zu dem Polyurethansystem, insbesondere Polyurethanschaumstoff, vorzugsweise in einer Menge von 0,0001 bis 10 Gew.-%, vorzugsweise 0,001 bis 5 Gew.-%, insbesondere 0,01 bis 3 Gew.-%, bezogen auf das Gesamtgewicht des Polyurethansystems, wobei die Zugabe vor, während oder nach der Herstellung des Polyurethansystems erfolgen kann. Die Verbindung der Formel (I) gelangt insbesondere als erfindungsgemäße Antioxidationsmittelmischung zum Einsatz, umfassend Verbindungen der Formel (I) sowie vorzugsweise Verbindungen der Formel (II), insbesondere umfassend Verbindungen der Formel (I) sowie Verbindungen der Formel (II) und (III),

Der Gegenstand der vorliegenden Erfindung wird nachfolgend an Hand von Beispielen näher erläutert, ohne dass der Gegenstand der Erfindung auf diese beispielhaften Ausführungsformen beschränkt sein soll.

### Beispiele

### Herstellung des erfindungsgemäßen Additivs der Formel (I)

Die Hydroxyphenylcarbonsäureester wurden nach dem oben beschriebenen Verfahren A ausgehend von Methyl-3-(3,5-di-tert-butyl-4-hydroxyphenyl)propionat (IVa-1) hergestellt.

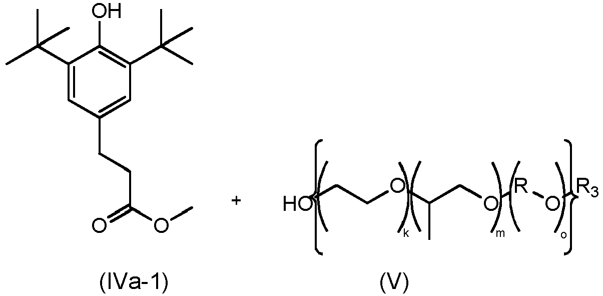

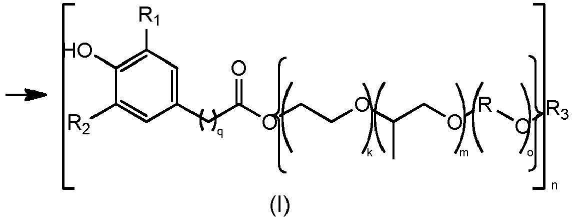

### Beispiel 1 (erfindungsgemäß)

In einen 250-mL-Dreihalskolben mit Destillationsbrücke und KPG-Rührer wurden 29,3 g des Hydroxylphenylcarbonsäureesters Methyl-3-(3,5-di-tert-butyl-4-hydroxyphenyl)propionat (IVa-1) zusammen mit 61,9 g eines Polyethers der allgemeinen Formel CH₃[CH₂CH₂O]₁₁H und 0,06 g Natriumacetat vorgelegt. Der Kolben wurde mit Stickstoff geflutet und die Mischung wurde auf 180 °C geheizt und eine Stunde gerührt. Anschließend wurde der Kolben evakuiert und das entstandene Methanol unter Rühren unter Vakuum (10 mbar) über eine Destillationsbrücke direkt aus dem Reaktionsgemisch abdestilliert. Nach dem Abkühlen wurden 6,9 g des Benzofuran-2-ons (IIa) und 1,7 g des Phosphits (IIIa) in dem erhaltenen flüssigen Hydroxyphenylcarbonsäureester gelöst.

### Beispiel 2 (erfindungsgemäß)

In einen 250-mL-Dreihalskolben mit Destillationsbrücke und KPG-Rührer wurden 29,3 g des Hydroxyphenylcarbonsäureesters Methyl-3-(3,5-di-tert-butyl-4-hydroxyphenyl)propionat (IVa-1) zusammen mit 75,8 g eines Polyethers der allgemeinen Formel CH₃[CH₂CH(CH₃)O]₂[CH₂CH₂O]₁₁H und 0,55 g Titantetrabutanolat vorgelegt. Der Kolben wurde mit Stickstoff geflutet und die Mischung wurde auf 180 °C geheizt und eine Stunde gerührt. Anschließend wurde der Kolben evakuiert und das entstandene Methanol unter Rühren unter Vakuum (10 mbar) über eine Destillationsbrücke direkt aus dem Reaktionsgemisch abdestilliert. Nach dem Abkühlen wurden 7,9 g des Benzofuran-2-ons (IIa) und 2,0 g des Phosphits (IIIa) in dem erhaltenen flüssigen Hydroxyphenylcarbonsäureester gelöst.

### Beispiel 3 (nicht erfindungsgemäß)

In einen 250-mL-Dreihalskolben mit Destillationsbrücke und KPG-Rührer wurden 29,3 g des Hydroxyphenylcarbonsäureesters Methyl-3-(3,5-di-tert-butyl-4-hydroxyphenyl)propionat (IVa-1) zusammen mit 15,6 g 1-Oktanol und 0,2 g para-Toluolsulfonsäure vorgelegt. Der Kolben wurde mit Stickstoff geflutet und die Mischung wurde auf 180 °C geheizt und eine Stunde gerührt. Anschließend wurde der Kolben evakuiert und das entstandene Methanol unter Rühren unter Vakuum (10 mbar) über eine Destillationsbrücke direkt aus dem Reaktionsgemisch abdestilliert. Nach dem Abkühlen wurden 3,5 g des Benzofuran-2-ons (IIa) und 0,9 g des Phosphits (IIIa) in dem erhaltenen flüssigen Hydroxyphenylcarbonsäureester gelöst.

### Herstellung der Polyurethanschäume

Bei den anwendungstechnischen Tests wurden drei typische Formulierungen für Polyurethanweichschäume, die sich wie folgt zusammensetzen, verwendet:

**Tabelle 1: Formulierung I für TDI80-Weichblockschaum-Anwendungen zur Ausprüfung der Anti-Scorch Performance (Scorch = Degradation des Polyurethans)**

| **Formulierung I** | **Massenteile (pphp)** |
|---|---|
| Voranol® CP 3322¹⁾ | 100 |
| Desmodur® T 80²⁾ Index <104> | 56,4 |
| Wasser | 4,8 |
| TEGOAMIN® 33³⁾ | 0,2 |
| KOSMOS® 29⁴⁾ | 0,22 |
| Fyrol™ A300TB⁵⁾ | 10,0 |
| TEGOSTAB® B 8242⁶⁾ | 1,0 |
| Antioxidationsmittelmischung⁷⁾ | 1,0 |

| | |
|---|---|
| ¹⁾ erhältlich bei der Firma Dow Chemical; hierbei handelt es sich um ein Glycerin-basiertes Polyetherpolyol mit einer OH-Zahl von 48 mg KOH/g. ²⁾ Toluylendiisocyanat T 80 (80 % 2,4-Isomer, 20 % 2,6-Isomer) der Firma Bayer Material Science, 3 mPa·s, 48 % NCO, Funktionalität 2. ³⁾ Amin-Katalysator der Firma Evonik Industries AG. ⁴⁾ Zinnkatalysator, erhältlich bei der Firma Evonik Industries AG. ⁵⁾ Phosphorbasiertes Flammschutzmittel der Firma ICL Industrial Products. ⁶⁾ Polyethermodifiziertes Polysiloxan, erhältlich bei der Firma Evonik Industries AG. ⁷⁾ Erfindungsgemäße Antioxidationsmittelmischung, dargestellt nach den Beispielen 1 - 2, nicht erfindungsgemäße Antioxidationsmittelmischung, dargestellt nach Beispiel 3 oder Antioxidationsmittelmischung ORTEGOL® AO 5 der Firma Evonik Industries AG. | |

**Tabelle 2: Formulierung II für TDI80-Weichblockschaum-Anwendungen zur Bestimmung der VOC- und Fog-Emissionen gemäß DaimlerChrysler Prüfanweisung VDA 278**

| **Formulierung II** | **Massenteile (pphp)** |
|---|---|
| Arcol® 1105 S⁸⁾ | 100 |
| Desmodur® T 80²⁾ Index <110> | 41,6 |
| Wasser | 3,0 |
| TEGOAMIN® ZE1³⁾ | 0,15 |
| KOSMOS® EF⁴⁾ | 0,6 |
| TEGOSTAB® BF 2370⁶⁾ | 0,8 |
| Antioxidationsmittelmischung⁷⁾ | 1,0 |

| | |
|---|---|
| ²⁾ Toluylendiisocyanat T 80 (80 % 2,4-Isomer, 20 % 2,6-Isomer) der Firma Bayer Material Science, 3 mPa·s, 48 % NCO, Funktionalität 2. ³⁾ Amin-Katalysator der Firma Evonik Industries AG. ⁴⁾ Zinnkatalysator, erhältlich bei der Firma Evonik Industries AG ⁶⁾ Polyethermodifiziertes Polysiloxan, erhältlich bei der Firma Evonik Industries AG. ⁷⁾ Erfindungsgemäße Antioxidationsmittelmischung, dargestellt nach den Beispielen 1 - 2, nicht erfindungsgemäße Antioxidationsmittelmischung, dargestellt nach Beispiel 3 oder Antioxidationsmittelmischung ORTEGOL® AO 5 der Firma Evonik Industries AG. ⁸⁾ erhältlich bei der Firma Bayer Material Science; hierbei handelt es sich um ein Glycerin-basiertes Polyetherpolyol mit einer OH-Zahl von 56 mg KOH/g. | |

**Tabelle 3: Formulierung III für TDI80-Weichblockschaum-Anwendungen zur Bestimmung der VOC- und Fog-Emissionen gemäß DaimlerChrysler Prüfanweisung VDA 278**

| **Formulierung III** | **Massenteile (pphp)** |
|---|---|
| Arcol® 1105 S⁸⁾ | 100 |
| Desmodur® T 80²⁾ Index <110> | 62,9 |
| Wasser | 5,0 |
| TEGOAMIN® ZE1³⁾ | 0,15 |
| KOSMOS® EF⁴⁾ | 0,6 |
| TEGOSTAB® BF 2370⁶⁾ | 1,0 |
| Antioxidationsmittelmischung⁷⁾ | 1,0 |

| | |
|---|---|
| ²⁾ Toluylendiisocyanat T 80 (80 % 2,4-Isomer, 20 % 2,6-Isomer) der Firma Bayer Material Science, 3 mPa·s, 48 % NCO, Funktionalität 2. ³⁾ Amin-Katalysator der Firma Evonik Industries AG. ⁴⁾ Zinnkatalysator, erhältlich bei der Firma Evonik Industries AG ⁶⁾ Polyethermodifiziertes Polysiloxan, erhältlich bei der Firma Evonik Industries AG. ⁷⁾ Erfindungsgemäße Antioxidationsmittelmischung, dargestellt nach den Beispielen 1 - 2, nicht erfindungsgemäße Antioxidationsmittelmischung, dargestellt nach Beispiel 3 oder Antioxidationsmittelmischung ORTEGOL® AO 5 der Firma Evonik Industries AG. ⁸⁾ erhältlich bei der Firma Bayer Material Science; hierbei handelt es sich um ein Glycerin-basiertes Polyetherpolyol mit einer OH-Zahl von 56 mg KOH/g. | |

### Allgemeine Durchführung zur Herstellung der Schäume

Die Herstellung der Schäume erfolgte nach folgenden Angaben bei 22 °C und 753 mm Hg Luftdruck. Zur Herstellung der Polyurethanschäume für den Mikrowellentest wurden jeweils 100 g Polyol eingesetzt, zur Herstellung der Polyurethanschäume für den Ofentest und die geruchliche Überprüfung wurden jeweils 300 g Polyol eingesetzt, zur Herstellung der Polyurethanschäume zur Bestimmung der VOC- und Fog-Emissionen wurden jeweils 500 g Polyol eingesetzt; die anderen Formulierungsbestandteile wurden entsprechend umgerechnet. Dabei bedeutete beispielsweise 1,0 Teil (1,0 pphp) einer Komponente 1 g dieser Substanz je 100 g Polyol.

Zur Verschäumung wurden das Polyol, Wasser, Katalysator (Amin(e) und/oder die Zinnverbindung), Stabilisator und die verwendete Antioxidationsmittelmischung unter Rühren gut vermischt. Nach Zugabe des Isocyanats wurde mit einem Rührer 7 Sek. bei 3000 U/Min. gerührt und das Gemisch in einen mit Papier ausgekleideten Holzkasten gegossen. Entstandene Polyurethanweichschäume wurden den nachfolgend beschriebenen anwendungstechnischen Tests unterzogen.

Zur Demonstration der Anti-Scorch Performance der vorliegenden Erfindung wurde eine Formulierung gewählt, die durch Wasser getrieben und frei gestiegen (Schaum kann ungehindert aufsteigen; keine Formschäume) ist. Die Wassermenge wurde mit 4,8 Teilen pro 100 Teile Polyolmischung gewählt. Aufgrund dieser Wassermenge kann ein Raumgewicht von etwa 21 kg/m³ erwartet werden. Somit ist die Formulierung hinsichtlich Raumgewicht und Wassermenge typisch für Polyurethanweichschaumqualitäten, die derzeit in der Industrie eingesetzt werden.

Zur Bestimmung der VOC- und Fog-Emissionen wurden zwei verschiedene Formulierungen gewählt, wobei beide jedoch durch Wasser getrieben und frei gestiegen (Schaum kann ungehindert aufsteigen; keine Formschäume) sind. Im ersten Fall wurde die Wassermenge mit 3 Teilen pro 100 Teile Polyol gewählt, im zweiten Fall wurden 5 Teile Wasser pro 100 Teile Polyol eingesetzt. So konnten Schäume mit Raumgewichten von etwa 30 kg/m³ bzw. 17 kg/m³ erhalten werden. Durch die verschiedenen Wassergehalte in den Formulierungen sollten unterschiedliche Temperaturen während des Verschäumungsprozesses erzeugt werden und so der Einfluss der Temperatur im Schaum auf die Emissionen untersucht werden.

### Anwendungstechnische Tests

Die hergestellten Schäume wurden anhand folgender physikalischer Eigenschaften bewertet:
a) Rücksacken des Schaumstoffes nach dem Ende der Steigphase (=Rückfall).
   Der Rückfall, bzw. das Nachsteigen ergibt sich aus der Differenz der Schaumhöhe nach direktem Abblasen und nach 3 min. nach Abblasen des Schaums. Die Schaumhöhe wird dabei durch eine an einem Zentimetermaß befestigte Nadel auf dem Maximum in der Mitte der Schaumkuppe gemessen. Ein negativer Wert beschreibt hierbei das Rücksacken des Schaumes nach dem Abblasen, ein positiver Wert beschreibt entsprechend das Nachsteigen des Schaumes.
b) Schaumhöhe ist die Höhe des gebildeten, frei gestiegenen Schaums nach 3 Minuten. Die Schaumhöhe wird in Zentimeter (cm) angegeben.
c) Steigzeit
   Die Zeitspanne zwischen dem Ende des Mischens der Reaktionskomponenten und dem Abblasen des Polyurethanschaums.
d) Raumgewicht
   Die Bestimmung erfolgt, wie in DIN EN ISO 845:2009-10 beschrieben, durch Messung der Rohdichte. Das Raumgewicht wird in kg/m³ angegeben.
e) Porosität
   Die Luftdurchlässigkeit des Schaums wurde in Anlehnung an DIN EN ISO 4638:1993-07 durch eine Staudruckmessung am Schaumstoff ermittelt. Der gemessene Staudruck wurde in mm Wassersäule angegeben, wobei dann die niedrigeren Staudruckwerte den offeneren Schaum charakterisieren. Die Werte wurden im Bereich von 0 bis 300 mm gemessen. Die Messung des Staudrucks erfolgte mittels einer Apparatur umfassend eine Stickstoffquelle, Reduzierventil mit Manometer, Durchflussregelschraube, Waschflasche, Durchflussmessgerät, T-Stück, Auflagedüse und einem skaliertem Glasrohr, in welches Wasser gefüllt ist. Die Auflagedüse weist eine Kantenlänge von 100 x 100 mm, ein Gewicht von 800 g, eine lichte Weite der Austrittsöffnung von 5 mm, eine lichte Weite des unteren Auflageringes von 20 mm und einen Außendurchmesser des unteren Auflageringes von 30 mm auf.
   Die Messung erfolgt durch Einstellung des Stickstoffvordrucks per Reduzierventil auf 1 bar und Einregeln der Durchflussmenge auf 480 l/h. Die Wassermenge wird im skalierten Glasrohr so eingestellt, dass keine Druckdifferenz aufgebaut und ablesbar ist. Für die Vermessung des Prüfkörpers mit einer Dimension von 250 x 250 x 50 mm wird die Auflagedüse an den Ecken des Prüfkörpers kantenkongruent aufgelegt sowie einmal an der (geschätzten) Mitte des Prüfkörpers (jeweils auf der Seite mit der größten Oberfläche) aufgelegt. Abgelesen wird, wenn sich ein konstanter Staudruck eingestellt hat.
   Die Auswertung erfolgt durch Mittelwertbildung über die fünf erhaltenen Messwerte.

### Messung der Schaum-Emissionen (VOC- und Fog-Wert) in Anlehnung an die Prüfvorschrift VDA 278 in der Version vom Oktober 2011:

Das Verfahren dient zur Ermittlung von Emissionen aus nichtmetallischen Materialien, die für Formteile in Kraftfahrzeugen zum Einsatz kommen. Die Emission von leichtflüchtigen organischen Verbindungen (VOC-Wert, 30 Minuten bei 90 °C) sowie dem Anteil kondensierbarer Substanzen (Fog-Wert, 60 Minuten bei 120 °C) wurde in Anlehnung an die Prüfvorschrift VDA 278 in der Version vom Oktober 2011 bestimmt. Im Folgenden wird die Durchführung der entsprechenden Thermodesorption mit anschließender Gaschromatographie/Massenspektrometrie-Kopplung (GC/MS) beschrieben.
a) Messtechnik: Die Thermodesorption wurde mit einem Thermodesorber "TDS2" mit Probenwechsler der Fa. Gerstel, Mülheim, in Verbindung mit einem Agilent 7890/5975 GC/MSD-System durchgeführt.
b) Die Messbedingungen für VOC-Messungen sind in Tabellen 4 und 5 angegeben.

**Tabelle 4: Messparameter Thermodesorption für den VOC-Analysenlauf.**

| | |
|---|---|
| Thermodesorption | Gerstel TDS2 |
| Desorptionstemperatur | 90 °C |
| Desorptionsdauer | 30 min |
| Fluss | 65 ml/min |
| Transferline | 280°C |
| Kryofokussierung | KAS 4 |
| Liner | Glasverdampferrohr mit silanisierter Glaswolle |
| Temperatur | -150°C |

**Tabelle 5: Messparameter Gaschromatographie/Massenspektrometrie für den VOC-Analysenlauf.**

| | |
|---|---|
| GC | Kapillar-GC Agilent 7890 |
| Injektor | PTV Split 1:50 |
| Temperaturprogramm | -150°C; 1 min; 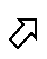 10°C/s; 280°C |
| Säule | Agilent 19091B-115, Ultra 2, 50 m * 0,32 mm dF 0,5µm |
| Fluss | 1,3 ml/min const. Flow |
| Temperaturprogramm | 50°C; 2 min; 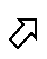 3°C/min; 92°C; 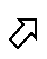 5°C/min; 160°C; 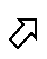 10°C/min; 280°C, 20 min |
| Detektor | Agilent MSD 5975 |
| Modus | Scan 29-350 amu 2,3 scans/sec |
| Auswertung | Auswertung des Totalionenstrom-Chromatrogramms durch Berechnung als Toluoläquivalent |

c) Kalibrierung: Zur Kalibrierung wurde 2 µl eines Gemisches aus Toluol und Hexadekan in Methanol (je 0,125 mg/ml) auf ein gereinigtes, mit Tenax® TA (mesh 35/60) gefülltes Adsorptionsröhrchen gegeben und vermessen (Desorption 5 min; 280 °C).
d) Bei Tenax® TA handelt es sich um ein poröses Polymerharz basierend auf 2.6-Diphenylenoxid, erhältlich beispielsweise bei der Firma Scientific Instrument Services, 1027 Old York Rd., Ringoes, NJ 08551.
e) Probenvorbereitung für die VOC-Messung: 15 mg Schaumstoff wurden in drei Teilproben in einem Thermodesorptionsröhrchen platziert. Dabei wurde darauf geachtet, dass der Schaum nicht komprimiert wird.
f) Probenvorbereitung für die Fog-Messung: Es wurde die gleiche Schaumprobe verwendet, wie für die VOC-Messung. Hinsichtlich der Messanordnung wurde immer zuerst die VOC-Messung und im Anschluss die Fog-Messung durchgeführt, wobei mittels eines Autosampler-Systems ein jeweils konstanter Abstand zwischen den entsprechenden VOC- und Fog-Messungen gewährleistet wurde.
g) Die Messbedingungen für Fog-Messungen sind in Tabelle 6 und 7 wiedergegeben.

**Tabelle 6: Messparameter Thermodesorption für den Fog-Analysenlauf.**

| | |
|---|---|
| Thermodesorption | Gerstel TDS2 |
| Desorptionstemperatur | 120 °C |
| Desorptionsdauer | 60 min |
| Fluss | 65 ml/min |
| Transferline | 280°C |
| Kryofokussierung | KAS 4 |
| Liner | Glasverdampferrohr mit silanisierter Glaswolle |
| Temperatur | -150°C |

**Tabelle 7: Messparameter Gaschromatographie/Massenspektrometrie für den Fog-Analysenlauf.**

| | |
|---|---|
| GC | Kapillar-GC Agilent 7890 |
| Injektor | PTV Split 1:50 |
| Temperaturprogramm | -150°C; 1 min; 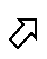 10°C/s; 280°C |
| Säule | Agilent 19091B-115, Ultra 2, 50 m * 0,32 mm dF 0,5µm |
| Fluss | 1,3 ml/min const. Flow |
| Temperaturprogramm | 50°C; 2 min; 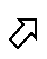 25°C/min; 160°C; 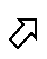 10°C/min; 280°C; 20 min |
| Detektor | Agilent MSD 5975 |
| Modus | Scan 29-450 amu 2,3 scans/sec |
| Auswertung | Auswertung des Totalionenstrom-Chromatrogramms durch Berechnung als Hexadekanäquivalent |

h) Kalibrierung: Zur Kalibrierung wurde 2 µl eines Gemisches aus Toluol und Hexadekan in Methanol (je 0,125 mg/ml) auf ein gereinigtes, mit Tenax® TA (mesh 35/60) gefülltes Adsorptionsröhrchen gegeben und vermessen (Desorption 5 min; 280 °C).

Für die Messung der Emissionen wurden Schäume verwendet, welche nach den Formulierungen II und III und unter Einsatz von 500 g Polyol dargestellt wurden.

### Überprüfung der Anti-Scorch Performance

### a) Mikrowellentest

Die Schäume, welche durch Einsatz von 100 g Polyol durch Umsatz nach Formulierung I erhalten wurden, wurden drei Minuten, nachdem die flüssige Mischung in den mit Papier ausgekleideten Holzkasten gegossen wurde, im Mikrowellenofen für 80 s bei 1000 W bestrahlt. Anschließend wurden die Schäume vertikal in der Mitte durchgeschnitten und die Kernverfärbung optisch begutachtet. Eine leichte Kernverfärbung wurde mit +, eine mittlere Kernverfärbung mit ++ und eine starke Kernverfärbung mit +++ bewertet. Keine erkennbare Verfärbung wurde mit - bewertet.

### b) Ofentest

Die Schäume, welche durch Einsatz von 300 g Polyol durch Umsatz nach Formulierung I erhalten wurden, wurden 3 Minuten nach dem Ende der Steigzeit für 5 Minuten bei 150°C in einen Trockenschrank gegeben. Anschließend wurde das Papier entfernt und der Schaum für weitere 2 Stunden bei 180°C erwärmt. Es wird zunächst von unten eine 3 cm Schicht abgeschnitten. Bei der folgende 5 cm Schicht wird die Kernverfärbung optisch beurteilt. Eine leichte Kernverfärbung wurde mit +, eine mittlere Kernverfärbung mit ++ und eine starke Kernverfärbung mit +++ bewertet. Keine erkennbare Verfärbung wurde mit - bewertet.

### Geruchliche Überprüfung der erhaltenen Schäume

Die fertigen Schäume, dargestellt aus 300 g Polyol nach Formulierung I wurden in geruchsneutralen Kunststofftüten gepackt und luftdicht aufbewahrt. Für die Geruchsbewertung des Schaums wurden Würfel der Größe 10 cm x 10 cm x 10 cm ausgeschnitten und in Gläser mit einem Volumen von 1 L überführt, aus denen die Proben abgerochen wurden. Die Gläser wurden mit einem Schraubdeckel verschlossen. Die geruchliche Überprüfung erfolgte nach 24 stündiger Lagerung der Gläser bei 22°C.

Die geruchliche Überprüfung wurde von einem trainierten Panel mit einer Anzahl von 10 Personen bewertet. Hierbei wurde die Intensität des Geruchs abgefragt, wenig Geruch wurde mit +, mittelstarker Geruch mit ++ und starker Geruch wurde mit +++ bewertet.

### Ergebnisse der Verschäumungen

Die erfindungsgemäßen Additive der Beispiele 1 und 2, das in Beispiel 3 beschriebene, nicht erfindungsgemäße Additiv sowie die kommerziell erhältliche Antioxidationsmittelmischung ORTEGOL® AO 5 der Firma Evonik Industries AG wurden hinsichtlich ihrer Eigenschaft die Kernverfärbung während des Verschäumprozesses zu inhibieren in Formulierung I ausgetestet und die erhaltenen Schäume wie oben beschrieben entweder in der Mikrowelle bestrahlt oder im Ofen erhitzt. Anschließend wurden die Schäume aufgeschnitten und die Kernverfärbung optisch beurteilt.

In Hinsicht auf die Emissionen wurden die erfindungsgemäßen Additive der Beispiele 1 und 2, das in Beispiel 3 beschriebene, nicht erfindungsgemäße Additiv sowie die kommerziell erhältliche Antioxidationsmittelmischung ORTEGOL® AO 5 der Firma Evonik Industries AG in den Formulierungen II und III verschäumt und die VOC- und Fog-Emissionen wie oben beschrieben nach VDA 278 (Oktober 2011) ermittelt.

Die Ergebnisse sind in den folgenden Tabellen 8 - 11 wiedergegeben:
Wie in Tabelle 8 dargestellt, werden ohne den Einsatz eines Antioxidationsmittels mit Formulierung I Polyurethanweichschäume erhalten, welche in beiden Scorchingtests (Mikrowellen- und Ofentest) eine starke Kernverfärbung (Tabelle 8, Eintrag 1) aufweisen. Beim Einsatz von 1 pphp der Vergleichsantioxidationsmittelmischung ORTEGOL® AO 5 der Firma Evonik Industries AG wurde im Mikrowellentest eine mittelstarke und im Ofentest eine leichte Kernverfärbung beobachtet (Tabelle 8, Eintrag 2). Die nicht erfindungsgemäße Antioxidationsmittelmischung, dargestellt nach Beispiel 3, lieferte ebenfalls Polyurethanweichschäume mit mittelstarker Kernverfärbung im Mikrowellentest und eine leichter Kernverfärbung im Ofentest (Tabelle 8, Eintrag 5). Durch Einsatz von 1 pphp der erfindungsgemäßen Antioxidationsmittelmischungen (Beispiel 1 und 2) konnten sowohl im Mikrowellentest als auch im Ofentest verbesserte Verfärbungswerte beobachtet werden (Tabelle 8, Eintrag 3 und 4). Schaumstoffe, gekennzeichnet durch den Einsatz von 1 pphp der Antioxidationsmittelmischungen ORTEGOL® AO 5 oder der nicht erfindungsgemäßen Antioxidationsmittelmischung, dargestellt nach Beispiel 3, weisen extrem hohe Emissionswerte auf, wohingegen Schaumstoffe, dargestellt nach Einsatz von 1 pphp der erfindungsgemäßen Antioxidationsmittelmischungen nach den Beispielen 1 und 2, sehr niedrige VOC- und Fog-Emissionswerte lieferten. Diese Schaumstoffe wurden nach Formulierung II (3 pphp H₂O) bzw. nach Formulierung III (5 pphp H₂O) dargestellt und die Emissionen in Anlehnung an VDA 278 ermittelt. Es waren keine signifikanten Unterschiede der einzelnen Antioxidationsmittelmischungen in den beiden verschiedenen Formulierungen zu erkennen, sodass der Einfluss verschiedener Temperaturen während des Verschäumungsprozesses auf das Emissionsverhalten als vernachlässigbar betrachtet werden kann. Es konnte jedoch gezeigt werden, dass die erfindungsgemäßen Additivmischungen sowohl im VOC- als auch im Fog-Bereich weitaus geringere Emissionen aufwiesen (Tabelle 9, Eintrag 7 und 8; Tabelle 10, Eintrag 11 und 12) als die kommerziell erhältliche Antioxidationsmittelmischung ORTEGOL® AO 5 und die in Beispiel 3 dargestellte nicht erfindungsgemäße Antioxidationsmittelmischung (Tabelle 9, Eintrag 6 und 9; Tabelle 10, Eintrag 10 und 13).

Wie Tabelle 11 zeigt, wurde die Intensität des Geruches der Schäume, welche mit den erfindungsgemäßen Additiven aus den Beispielen 1 und 2 dargestellt wurden (Einträge 15 - 16), durchweg geringer beurteilt als der Geruch des Schaumstoffs, welcher mit der Vergleichsantioxidationsmittelmischung ORTEGOL® AO 5 der Firma Evonik Industries AG hergestellt wurde (Eintrag 14). Ebenso wies der Schaumstoff, hergestellt mit der Antioxidationsmittelmischung nach Beispiel 3 (nicht erfindungsgemäß), einen stärkeren Geruch auf als die Schaumstoffe dargestellt mit den beiden erfindungsgemäßen Antioxidationsmittelmischungen. Die geruchliche Überprüfung wurde von den Probanden noch zweimal wiederholt, wobei die vorgenannten Ergebnisse in genau der gleichen Weise bestätigt wurden. Aus den Ergebnissen ist ersichtlich, dass die Probanden einen Schaum, der mit einem der erfindungsgemäßen Additivmischungen behandelt wurde, als weniger intensiv riechend beurteilt haben.

**Tabelle 8: Verschäumungsergebnisse und Kernverfärbung bei Verwendung verschiedener Antioxidationsmittel nach Formulierung I**

| Nr. | Additiv | Einsatzmenge [pphp] | Steigzeit [s] | Steighöhe [cm] | Rückfall [cm] | Porosität [mm] | Dichte [kg/m³] | Kernverfärbung Mikrowellentest | Kernverfärbung Ofentest |
|---|---|---|---|---|---|---|---|---|---|
| 1 | Referenz | 0 | 96 | 21,5 | 0,2 | 9 | 20,7 | +++ | +++ |
| 2 | ORTEGOL® AO 5 ^{a)} | 1 | 94 | 20,3 | 0,1 | 10 | 21,2 | ++ | + |
| 3 | Bsp. 1 ^{b)} | 1 | 98 | 21,0 | 0,2 | 8 | 21,1 | + | - |
| 4 | Bsp. 2 ^{b)} | 1 | 98 | 21,3 | 0,2 | 8 | 21,0 | + | - |
| 5 | Bsp. 3 ^{c)} | 1 | 96 | 21,0 | 0,2 | 7 | 21,1 | ++ | + |

| | | | | | | | | | |
|---|---|---|---|---|---|---|---|---|---|
| ^{a)} Vergleichsantioxidationsmittelmischung der Firma Evonik Industries AG ^{b)} erfindungsgemäße Additive, dargestellt nach den Beispielen 1 und 2 ^{c)} nicht erfindungsgemäßes Additiv, dargestellt nach Beispiel 3 - keine Verfärbung erkennbar + leichte Verfärbung ++ mittelstarke Verfärbung +++ starke Verfärbung | | | | | | | | | |

**Tabelle 9: Verschäumungsergebnisse und VOC- und Fog-Emissionen bei Verwendung verschiedener Antioxidationsmittel nach Formulierung II**

| Nr. | Additiv | Einsatzmenge [pphp] | Steigzeit [s] | Steighöhe [cm] | Rückfall [cm] | Porosität [mm] | Dichte [kg/m³] | VOC [µg/m³] | Fog [µg/m³] |
|---|---|---|---|---|---|---|---|---|---|
| 6 | ORTEGOL® AO 5 ^{a)} | 1 | 128 | 32,0 | 0,3 | 9 | 29,9 | 61 | 2030 |
| 7 | Bsp. 1 ^{b)} | 1 | 126 | 31,8 | 0,1 | 12 | 30,3 | 29 | 200 |
| 8 | Bsp. 2 ^{b)} | 1 | 126 | 31,7 | 0,2 | 16 | 30,2 | 30 | 171 |
| 9 | Bsp. 3 ^{c)} | 1 | 124 | 32,0 | 0,2 | 13 | 30,1 | 41 | 1467 |

| | | | | | | | | | |
|---|---|---|---|---|---|---|---|---|---|
| ^{a)} Vergleichsantioxidationsmittelmischung der Firma Evonik Industries AG ^{b)} erfindungsgemäße Additive, dargestellt nach den Beispielen 1 und 2 ^{c)} nicht erfindungsgemäßes Additiv, dargestellt nach Beispiel 3 | | | | | | | | | |

**Tabelle 10: Verschäumungsergebnisse und VOC- und Fog-Emissionen bei Verwendung verschiedener Antioxidationsmittel nach Formulierung III**

| Nr. | Additiv | Einsatzmenge [pphp] | Steigzeit [s] | Steighöhe [cm] | Rückfall [cm] | Porosität [mm] | Dichte [kg/m³] | VOC [µg/m³] | Fog [µg/m³] |
|---|---|---|---|---|---|---|---|---|---|
| 10 | ORTEGOL® AO 5 ^{a)} | 1 | 82 | 31,2 | 0,1 | 12 | 17,0 | 63 | 2022 |
| 11 | Bsp. 1 ^{b)} | 1 | 83 | 30,8 | 0,2 | 13 | 17,3 | 30 | 181 |
| 12 | Bsp. 2 ^{b)} | 1 | 81 | 30,9 | 0,1 | 15 | 17,2 | 30 | 145 |
| 13 | Bsp. 3 ^{c)} | 1 | 80 | 31,0 | 0,1 | 10 | 17,0 | 43 | 1411 |

| | | | | | | | | | |
|---|---|---|---|---|---|---|---|---|---|
| ^{a)} Vergleichsantioxidationsmittelmischung der Firma Evonik Industries AG ^{b)} erfindungsgemäße Additive, dargestellt nach den Beispielen 1 und 2 ^{c)} nicht erfindungsgemäßes Additiv, dargestellt nach Beispiel 3 | | | | | | | | | |

**Tabelle 11: Geruchliche Überprüfung der Schäume nach Formulierung I durch 10 olfaktorisch geschulte Personen**

| Nr. | Additiv | Einsatzmenge [pphp] | Intensität des Geruchs | | |
|---|---|---|---|---|---|
| | | | +++ | ++ | + |
| 14 | ORTEGOL® AO 5 ^{a)} | 1 | 2 | 6 | 2 |
| 15 | Bsp. 1 ^{b)} | 1 | 0 | 3 | 7 |
| 16 | Bsp. 2 ^{b)} | 1 | 1 | 4 | 5 |
| 17 | Bsp. 3 ^{c)} | 1 | 1 | 6 | 3 |

| | | | | | |
|---|---|---|---|---|---|
| ^{a)} Vergleichsantioxidationsmittelmischung der Firma Evonik Industries AG ^{b)} erfindungsgemäße Additive, dargestellt nach den Beispielen 1 und 2 ^{c)} nicht erfindungsgemäßes Additiv, dargestellt nach Beispiel 3 + leichter Geruch ++ mittelstarker Geruch +++ starker Geruch | | | | | |

## Patentansprüche

1. Antioxidationsmittelmischung, enthaltend zumindest eine Verbindung der Formel (I) 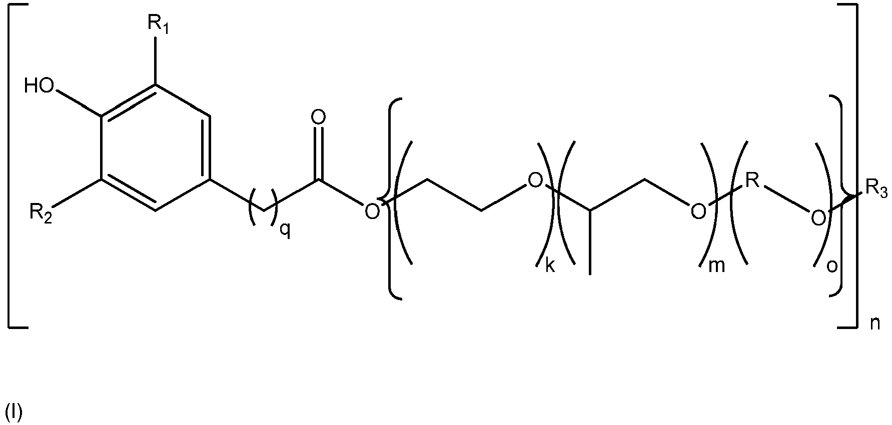 worin
R CH₂-CH(R^{I}), CH(R^{II})-CH(R^{II}), CH₂-C(R^{II})₂, C(R^{II})₂-C(R^{II})₂, 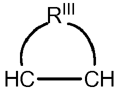 CH₂-CH-CH₂-R^{IV}, C₆H₆-CH-CH₂, oder C₆H₆-C(CH₃)-CH₂, mit
R^{I} C₂ bis C₂₄-Alkylrest oder Alkenrest, der linear oder verzweigt sein kann
R^{II}
C₂ bis C₂₄-Alkylrest oder Alkenrest, der linear oder verzweigt sein kann
R^{III} C₃ bis C₆ Alkylrest, der linear angeordnet ist, und
R^{IV} OH, Cl, OCH₃, OCH₂-CH₃, O-CH₂-CH=CH₂, O-CH=CH₂, Molekülrest von ein- oder mehrfach epoxidierten Fetten oder Ölen als Mono-, Di-, und Triglyceride oder Molekülrest von ein- oder mehrfach epoxidierten Fettsäuren oder deren C₁-C₂₄-Alkylestern,
R₁ und R₂ unabhängig voneinander geradkettig oder verzweigt C₁-C₈-Alkyl, Cyclopentyl oder Cyclohexyl, insbesondere tert.-Butyl,
q 1, 2 oder 3, vorzugsweise 2 oder 3, insbesondere 2,
n eine ganze Zahl von 1 bis 30, vorzugsweise eine ganze Zahl von 1 bis 10, vorteilhafterweise 1,2,3,4,5 oder 6, z.B. 1,2,3 oder 4, insbesondere 1,
R₃ einen n-wertigen Rest von linearem oder verzweigtem C₁-C₃₀₋Alkyl, C₂-C₃₀₋Alkylen, welche jeweils gegebenenfalls durch ein oder mehrere Sauerstoffatome unterbrochen sind, oder, insbesondere für n = 1-12, einen n-wertigen Rest von C₅-C₁₂-Cycloalkyl, oder einen Rest R₄-[NR₅-C_{q}H_{2q}-]ₚ, R₄ Wasserstoff, einen n-wertigen Rest von linearem oder verzweigtem C₁-C₃₀-Alkyl, welches gegebenenfalls durch eine oder mehrere Gruppen -NR₅- unterbrochen ist, oder, für n = 1-12, einen n-wertigen Rest von C₅-C₁₂-Cycloalkyl, R₅ unabhängig voneinander Wasserstoff oder Methyl oder -C_{q}H_{2q}-, vorzugsweise Wasserstoff, und p derjenigen Anzahl -[NR₅-C_{q}H_{2q}-]-Gruppen entspricht, welche n Reste -C_{q}H_{2q}- pro Molekül ergibt,
k eine ganze Zahl zwischen 0 und 50, vorzugsweise zwischen 10 und 30,
m eine ganze Zahl zwischen 0 und 50, z.B. 1-40, und
o eine ganze Zahl zwischen 0 und 50, vorzugsweise zwischen 0 und 30, insbesondere 0 darstellt,
mit (k + m + o) > 10.
und als weiteres Antioxidans
zumindest ein Benzofuranonderivat der Formel (II) 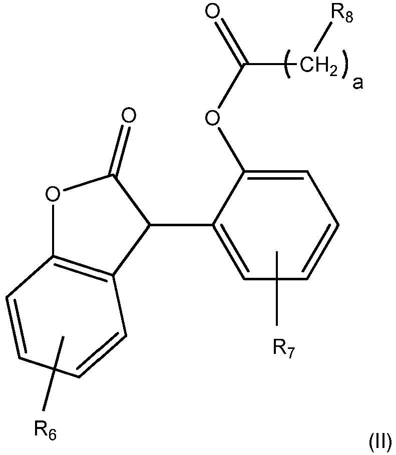 worin
a einer ganzen Zahl zwischen 0 und 7, vorzugsweise 0 - 3,
R₆ und R₇ unabhängig voneinander H oder C₁-C₈-Alkyl,
R₈ H oder einem aromatischen Rest 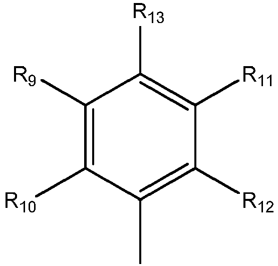
mit
R₉ und R₁₀ unabhängig voneinander H oder C₁-C₆-Alkyl, wobei nicht beide eine C₁-C₆-Alkyl darstellen,
R₁₁ und R₁₂ unabhängig voneinander H oder C₁-C₆-Alkyl, wobei nicht beide eine C₁-C₆-Alkyl darstellen,
R₁₃ H oder OH
entspricht.

2. Antioxidationsmittelmischung gemäß Anspruch 1, **dadurch gekennzeichnet, dass** die Verbindung der Formel (I) in einer Menge von 75 bis 99 Gew.-%, vorzugsweise 80 bis 98 Gew.-%, insbesondere 90 bis 95 Gew.-% und die Verbindung der Formel (II) in einer Menge von 1 bis 25 Gew.-%, vorzugsweise 2 bis 20 Gew.-%, insbesondere 5 bis 10 Gew.-% enthalten sind, Gew.-% bezogen auf das Gesamtgewicht der eingesetzten Verbindungen der Formeln (I) und (II).

3. Antioxidationsmittelmischung gemäß einem der Ansprüche 1 oder 2, **dadurch gekennzeichnet, dass** weiterhin ein Phosphit, insbesondere ein Phosphit der Formel (III), enthalten ist, 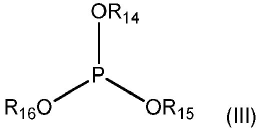 wobei
R₁₄, R₁₅ und R₁₆ unabhängig voneinander einen aromatischen oder aliphatischen, linearen oder verzweigten Rest von C₁-C₃₀₋Alkyl oder C₂-C₃₀₋Alkylen darstellen, welche jeweils gegebenenfalls durch ein oder mehrere Sauerstoffatome unterbrochen sind,
wobei das Phosphit vorzugsweise in Mengen von 0,1 bis 20 Gew.-%, vorzugsweise 0,2 bis 15 Gew.-% insbesondere 0,5 bis 10 Gew.-% enthalten ist,
Gew.-% bezogen auf das Gesamtgewicht der eingesetzten Verbindungen der Formeln (I), (II) und Phosphit.

4. Verfahren zur Herstellung von Polyurethan-Systemen durch Umsetzung mindestens einer Polyolkomponente mit mindestens einer Isocyanatkomponente in Gegenwart eines oder mehrerer Katalysatoren, die die Reaktionen Isocyanat-Polyol und/oder Isocyanat-Wasser und/oder die Isocyanat-Trimerisierung katalysieren, **dadurch gekennzeichnet, dass** die Umsetzung in Gegenwart einer Antioxidationsmittelmischung nach einem der Ansprüche 1 bis 3 durchgeführt wird.

5. Polyurethansystem, hergestellt gemäß einem Verfahren nach Anspruch 4, insbesondere enthaltend von 0,0001 bis 10 Gew.-%, vorzugsweise 0, 001 bis 5 Gew.-%, insbesondere 0,01 bis 3 Gew.-% einer oder mehrerer Verbindungen der Formel (I) bezogen auf das Gesamtgewicht des Polyurethansystems.

6. Verwendung von Polyurethansystemen gemäß Anspruch 5 als Kühlschrankisolierung, Dämmplatte, Sandwichelement, Rohrisolation, Sprühschaum, 1- & 1,5-Komponenten-Dosenschaum, Holzimitat, Modellschaum, Verpackungsschaum, Matratze, Möbelpolster, Werkstoff in Kraftfahrzeuginnenräumen, Automobil-Sitzpolster, Kopfstütze, Instrumententafel, Automobil-Innenverkleidung, Automobil-Dachhimmel, Schallabsorptionsmaterial, Lenkrad, Schuhsole, Teppichrückseitenschaum, Filterschaum, Dichtschaum, Dichtmittel und Kleber oder zur Herstellung entsprechender Produkte, insbesondere als Werkstoff in Kraftfahrzeuginnenräumen.

7. Verwendung von Antioxidationsmittelmischungen nach einem der Ansprüche 1 bis 3 zur Herstellung von emissionsarmen Polyurethansystemen mit einem verringerten Wert für VOC und das Fogging.

8. Verwendung von Antioxidationsmittelmischungen nach einem der Ansprüche 1 bis 3 zur Herstellung von geruchsarmen Polyurethansystemen.

## Claims

1. Antioxidant mixture comprising at least one compound of the formula (I) 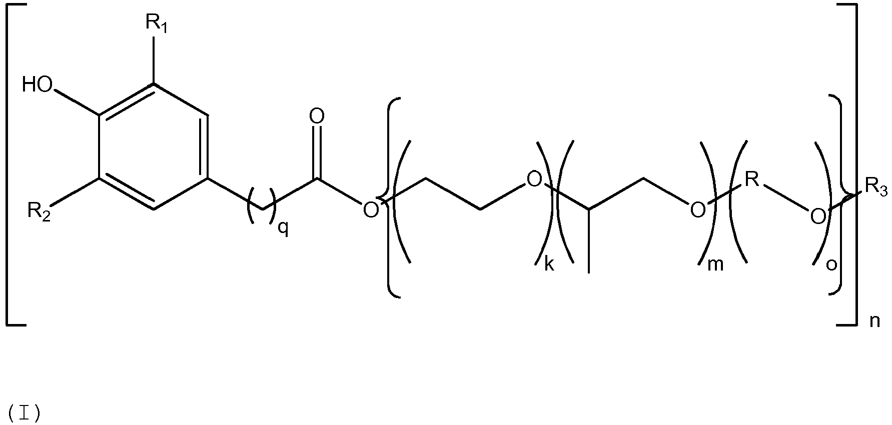 in which
R is CH₂-CH(R^{I}), CH(R^{II})-CH(R^{II}), CH₂-C(R^{II})₂, C(R^{II})₂-C(R^{II})₂, 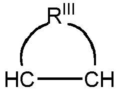 CH₂-CH-CH₂-R^{IV}, C₆H₆-CH-CH₂, or C₆H₆-C(CH₃)-CH₂,
where
R^{I} is C₂ to C₂₄ alkyl radical or alkene radical, which may be linear or branched
R^{II} is C₂ to C₂₄ alkyl radical or alkene radical, which may be linear or branched
R^{III} is C₃ to C₆ alkyl radical, which is arranged linearly, and
R^{IV} is OH, Cl, OCH₃, OCH₂-CH₃, O-CH₂-CH=CH₂, O-CH=CH₂,
molecule residue of singly or multiply epoxidized fats or oils as mono-, di-, and triglycerides, or molecule residue of singly or multiply epoxidized fatty acids or their C₁-C₂₄ alkyl esters,
R₁ and R₂ independently of one another are straight-chain or branched C₁-C₈ alkyl, cyclopentyl or cyclohexyl, especially tert-butyl,
q is 1, 2 or 3, preferably 2 or 3, especially 2,
n is an integer from 1 to 30, preferably an integer from 1 to 10, advantageously 1, 2, 3, 4, 5 or 6, e.g. 1, 2, 3 or 4, especially 1,
R₃ is an n-valent radical of linear or branched C₁-C₃₀-alkyl, C₂-C₃₀-alkylene, interrupted in each case optionally by one or more oxygen atoms, or, especially where n = 1-12, is an n-valent radical of C₅-C₁₂ cycloalkyl, or a radical R₄[NR₅-C_{q}H_{2q}-]ₚ,
R₄ is hydrogen, an n-valent radical of linear or branched C₁-C₃₀ alkyl, which is optionally interrupted by one or more groups -NR₅- or, where n = 1-12, is an n-valent radical of C₅-C₁₂ cycloalkyl,
R₅ independently at each occurrence is hydrogen or methyl or -C_{q}H_{2q}, preferably hydrogen, and
p corresponds to the number of -[NR₅-C_{q}H_{2q}-] groups that produces n radicals -C_{q}H_{2q}- per molecule,
k is an integer between 0 and 50, preferably between 10 and 30,
m is an integer between 0 and 50, e.g. 1-40, and
o is an integer between 0 and 50, preferably between 0 and 30, especially 0,
where (k + m + o) > 10,
and as further antioxidant
at least one benzofuranone derivative of the formula (II) 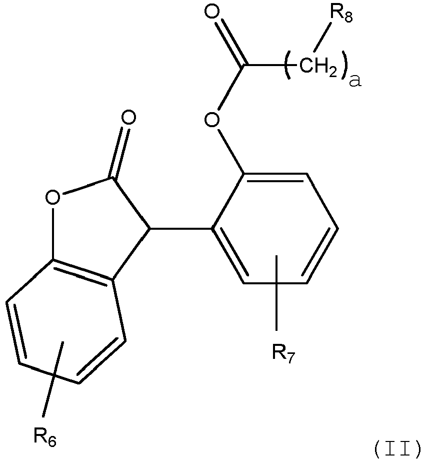 in which
a is an integer between 0 and 7, preferably 0-3,
R₆ and R₇ independently of one another are H or C₁-C₈ alkyl,
R₈ is H or an aromatic radical
where 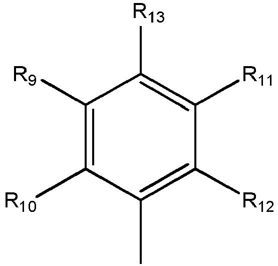
R₉ and R₁₀ independently of one another are H or C₁-C₆ alkyl, with not both being a C₁-C₆ alkyl,
R₁₁ and R₁₂ independently of one another are H or C₁-C₆ alkyl, with not both being a C₁-C₆ alkyl,
R₁₃ is H or OH.

2. Antioxidant mixture according to Claim 1, **characterized in that** the compound of the formula (I) is present in an amount of 75 to 99 wt%, preferably 80 to 98 wt%, more particularly 90 to 95 wt% and the compound of the formula (II) is present in an amount of 1 to 25 wt%, preferably 2 to 20 wt%, more particularly 5 to 10 wt%, wt% being based on the total weight of the compounds of the formulae (I) and (II) used.

3. Antioxidant mixture according to either of Claims 1 and 2, **characterized in that** a phosphite is further comprised, more particularly a phosphite of the formula (III), 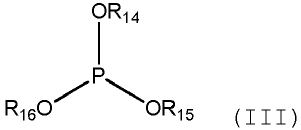 in which
R₁₄, R₁₅ and R₁₆ independently of one another are an aromatic or aliphatic, linear or branched radical of C₁-C₃₀ alkyl or C₂-C₃₀ alkylene, interrupted in each case optionally by one or more oxygen atoms,
the phosphite being present preferably in amounts of 0.1 to 20 wt%, preferably 0.2 to 15 wt%, more particularly 0.5 to 10 wt%, wt% being based on the total weight of the compounds of the formulae (I), (II) and phosphite used.

4. Process for producing polyurethane systems by reaction of at least one polyol component with at least one isocyanate component in the presence of one or more catalysts which catalyse the isocyanate-polyol and/or isocyanate-water reactions and/or the isocyanate trimerization, **characterized in that** the reaction is carried out in the presence of an antioxidant mixture according to any of Claims 1 to 3.

5. Polyurethane system produced by a process according to Claim 4,
more particularly comprising from 0.0001 to 10 wt%, preferably 0.001 to 5 wt%, more particularly 0.01 to 3 wt% of one or more compounds of the formula (I), based on the total weight of the polyurethane system.

6. Use of polyurethane systems according to Claim 5 as refrigerator insulation, insulant board, sandwich element, pipe insulation, sprayed foam, 1- and 1.5-component can foam, imitation wood, modelling foam, packaging foam, mattresses, furniture upholstery, material in motor vehicle interiors, vehicle seat upholstery, headrest, instrument panel, interior automotive trim, automotive roof liner, sound absorption material, steering wheel, footwear sole, carpet backing foam, filter foam, sealing foam, sealant and adhesive or for producing corresponding products, especially as material in motor vehicle interiors.

7. Use of antioxidant mixtures according to any of Claims 1 to 3 for producing low-emission polyurethane systems with reduced levels of VOC and fogging.

8. Use of antioxidant mixtures according to any of Claims 1 to 3 for producing low-odour polyurethane systems.

## Revendications

1. Mélange d'antioxydants, contenant au moins un composé de formule (I) 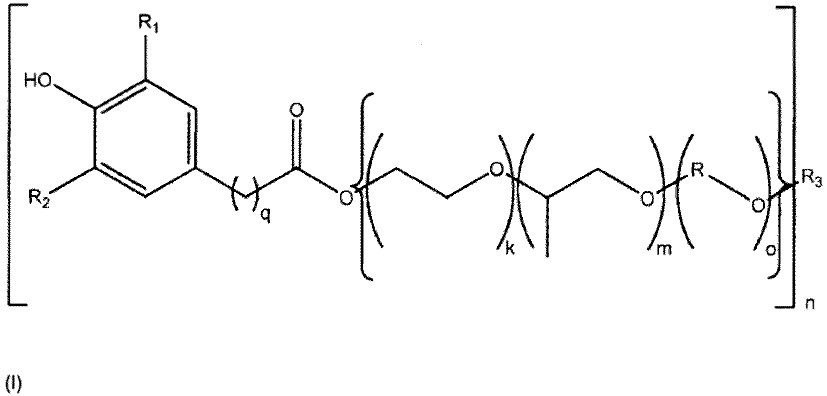 dans laquelle
R représente
CH₂-CH(R^{I}), CH(R^{II})-CH(R^{II}), CH₂-C(R^{II})₂, C(R^{II})₂-C(R^{II})₂, 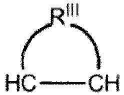 CH₂-CH-CH₂-R^{IV}, C₆H₆-CH-CH₂ ou C₆H₆-C(CH₃)-CH₂, avec
R^{I} radical alkyle en C₂ à C₂₄ ou radical alcène, qui peut être linéaire ou ramifié,
R^{II} radical alkyle en C₂ à C₂₄ ou radical alcène, qui peut être linéaire ou ramifié,
R^{III} radical alkyle en C₃ à C₆, qui est agencé linéairement, et
R^{IV} OH, Cl, OCH₃, OCH₂-CH₃, O-CH₂-CH=CH₂, O-CH=CH₂, radical moléculaire de matières grasses ou d'huiles mono- ou polyépoxydées en tant que mono-, di- et triglycérides ou radical moléculaire d'acides gras mono- ou polyépoxydés ou leurs esters alkyliques en C₁-C₂₄,
R₁ et R₂ représentent indépendamment l'un de l'autre, alkyle en C₁-C₈ linéaire ou ramifié, cyclopentyle ou cyclohexyle, notamment tert.-butyle,
q représente 1, 2 ou 3, de préférence 2 ou 3, notamment 2,
n représente un nombre entier de 1 à 30, de préférence un nombre entier de 1 à 10, avantageusement 1, 2, 3, 4, 5 ou 6, p. ex. 1, 2, 3 ou 4, notamment 1,
R₃ représente un radical n-valent d'alkyle en C₁-C₃₀ linéaire ou ramifié, alkylène en C₂-C₃₀, qui sont chacun éventuellement interrompus par un ou plusieurs atomes d'oxygène, ou, notamment pour n = 1 à 12, un radical n-valent de cycloalkyle en C₅-C₁₂, ou un radical R₄-[NR₅-C_{q}H_{2q}-]ₚ,
R₄ représente hydrogène, un radical n-valent d'alkyle en C₁-C₃₀ linéaire ou ramifié, qui est éventuellement interrompu par un ou plusieurs groupes -NR₅-, ou, pour n = 1 à 12, un radical n-valent de cycloalkyle en C₅-C₁₂, les R₅ représentent indépendamment les uns des autres hydrogène ou méthyle ou -C_{q}-H_{2q}-, de préférence hydrogène, et
p correspond au nombre de groupes -[NR₅-C_{q}H_{2q}-], qui résulte en n radicaux -C_{q}H_{2q}- par molécule,
k représente un nombre entier compris entre 0 et 50, de préférence entre 10 et 30,
m représente un nombre entier compris entre 0 et 50,
p. ex. 1 à 40, et
o représente un nombre entier compris entre 0 et 50, de préférence entre 0 et 30, notamment 0,
avec (k + m + o)> 10,
et en tant qu'oxydant supplémentaire,
au moins un dérivé de benzofuranone de formule (II) 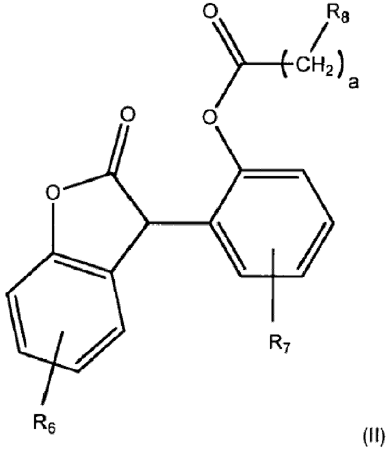 dans laquelle
a représente un nombre entier compris entre 0 et 7, de préférence 0 à 3,
R₆ et R₇ représentent indépendamment l'un de l'autre H ou alkyle en C₁-C₈, 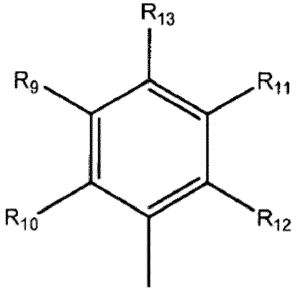
R₈ représente H ou un radical aromatique avec
R₉ et R₁₀ indépendamment l'un de l'autre H ou alkyle en C₁-C₆, les deux ne représentant pas un alkyle en C₁-C₆, R₁₁ et R₁₂ indépendamment l'un de l'autre H ou alkyle en C₁-C₆, les deux ne représentant pas un alkyle en C₁-C₆, R₁₃ H ou OH.

2. Mélange d'antioxydants selon la revendication 1, **caractérisé en ce que** le composé de formule (I) est contenu en une quantité de 75 à 99 % en poids, de préférence de 80 à 98 % en poids, notamment de 90 à 95 % en poids, et le composé de formule (II) en une quantité de 1 à 25 % en poids, de préférence de 2 à 20 % en poids, notamment de 5 à 10 % en poids, les % en poids se rapportant au poids total des composés de formule (I) et (II) utilisés.

3. Mélange d'antioxydants selon l'une quelconque des revendications 1 ou 2, **caractérisé en ce qu'**un phosphite, notamment un phosphite de formule (III), est également contenu : 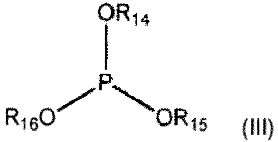 dans laquelle
R₁₄, R₁₅ et R₁₆ représentent indépendamment les uns des autres un radical aromatique ou aliphatique, linéaire ou ramifié, d'alkyle en C₁-C₃₀ ou d'alkylène en C₂-C₃₀, qui sont chacun éventuellement interrompus par un ou plusieurs atomes d'oxygène,
le phosphite étant de préférence contenu en quantités de 0,1 à 20 % en poids, de préférence de 0,2 à 15 % en poids, notamment de 0,5 à 10 % en poids,
les % en poids se rapportant au poids total des composés de formule (I), (II) et du phosphite utilisés.

4. Procédé de fabrication de systèmes de polyuréthane par mise en réaction d'au moins un composant polyol avec au moins un composant isocyanate en présence d'un ou de plusieurs catalyseurs qui catalysent les réactions isocyanate-polyol et/ou isocyanate-eau et/ou la trimérisation d'isocyanates, **caractérisé en ce que** la réaction est réalisée en présence d'un mélange d'antioxydants selon l'une quelconque des revendications 1 à 3.

5. Système de polyuréthane, fabriqué par un procédé selon la revendication 4, notamment contenant 0,0001 à 10 % en poids, de préférence 0,001 à 5 % en poids, notamment 0,01 à 3 % en poids d'un ou de plusieurs composés de formule (I) par rapport au poids total du système de polyuréthane.

6. Utilisation de systèmes de polyuréthane selon la revendication 5 en tant qu'isolation de réfrigérateur, panneau isolant, élément en sandwich, isolation de tube, mousse à pulvériser, mousse en boîte à 1 et 1,5 composant, imitation de bois, mousse de modèle, mousse d'emballage, matelas, rembourrage de meuble, matériau dans des espaces intérieurs de véhicules à moteur, rembourrage de siège automobile, appuie-tête, tableau de bord, habillage intérieur d'automobile, toit d'automobile, matériau d'absorption sonore, volant, semelle de chaussure, mousse pour le côté arrière de tapis, mousse filtrante, mousse d'étanchéité, agent d'étanchéité et colle ou pour la fabrication des produits correspondants, notamment en tant que matériau dans des espaces intérieurs de véhicules à moteur.

7. Utilisation de mélanges d'antioxydants selon l'une quelconque des revendications 1 à 3 pour la fabrication de systèmes de polyuréthane générant peu d'émissions ayant une valeur réduite pour les COV et le fogging.

8. Utilisation de mélanges d'antioxydants selon l'une quelconque des revendications 1 à 3 pour la fabrication de systèmes de polyuréthane générant peu d'odeurs.
